# EUROPÄISCHE PATENTSCHRIFT

(11) **EP 1 911 846 B1**
(45) Veröffentlichungstag und Bekanntmachung des Hinweises auf die Patenterteilung: **01.04.2015**
(21) Anmeldenummer: 07123951.1
(22) Anmeldetag: 11.11.2002
(51) Int. Cl.: C07K 14/415, C12N 9/04

(54) **Gene die für Glucose-6-Phosphat-Dehydrogenase Proteine codieren**
Genes which encode proteins for glucose-6-phosphate-dehydrogenase
Gène codant la protéine de glucose-6-phosphate-déshydrogénase

(30) Priorität: 13.11.2001 DE 10155505
(43) Veröffentlichungstag der Anmeldung: 16.04.2008
(62) Teilanmeldung aus: 02779545.9
(73) Patentinhaber: BASF SE, 67056 Ludwigshafen (DE)
(72) Erfinder: Zelder, Oskar, 67346 Speyer (DE); Pompejus, Markus, 67251 Freinsheim (DE); Schröder, Hartwig, 69226 Nußloch (DE); Kröger, Burkhard, 67117 Limburgerhof (DE); Klopprogge, Corinna, 68239 Mannheim (DE); Haberhauer, Gregor, 67117 Limburgerhof (DE)

(56) Entgegenhaltungen:
- WO-A-01/04322
- WO-A-01/70995
- DATABASE EMBL [Online] 24. Januar 2001 (2001-01-24), XP002230686 Database accession no. AX065117 -& DATABASE SWALL [Online] 1. Juni 2001 (2001-06-01), XP002232070 Database accession no. CAC25798 -& WO 01/00844 A (BASF AG) 4. Januar 2001 (2001-01-04)

## Beschreibung

### Hintergrund der Erfindung

Bestimmte Produkte und Nebenprodukte von natürlich-vorkommenden Stoffwechselprozessen in Zellen werden in vielen Industriezweigen verwendet, einschließlich der Nahrungsmittel-, Futtermittel-, Kosmetik- und pharmazeutischen Industrie. Diese Moleküle, die gemeinsam als "Feinchemikalien" bezeichnet werden, umfassen organische Säuren, sowohl proteinogene als auch nicht-proteinogene Aminosäuren, Nukleotide und Nukleoside, Lipide und Fettsäuren, Diole, Kohlehydrate, aromatische Verbindungen, Vitamine und Cofaktoren sowie Enzyme. Ihre Produktion erfolgt am besten mittels Anzucht von Bakterien im Großmaßstab, die entwickelt wurden, um große Mengen des jeweils gewünschten Moleküls zu produzieren und sezernieren. Ein für diesen Zweck besonders geeigneter Organismus ist *Corynebakterium glutamicum,* ein gram-positives, nicht-pathogenes Bakterium. Über Stammselektion ist eine Reihe von Mutantenstämmen entwickelt worden, die ein Sortiment wünschenswerter Verbindungen produzieren. Die Auswahl von Stämmen, die hinsichtlich der Produktion eines bestimmten Moleküls verbessert sind, ist jedoch ein zeitaufwendiges und schwieriges Verfahren.

### Zusammenfassung der Erfindung

Diese Erfindung stellt neuartige Nukleinsäuremoleküle bereit, die sich zur Identifizierung oder Klassifizierung von Corynebacterium glutamicum oder verwandten Bakterienarten verwenden lassen. C. glutamicum ist ein gram-positives, aerobes Bakterium, das in der Industrie für die Produktion im Großmaßstab einer Reihe von Feinchemikalien, und auch zum Abbau von Kohlenwasserstoffen (bspw. beim Überlaufen von Rohöl) und zur Oxidation von Terpenoiden gemeinhin verwendet wird. Die Nukleinsäuremoleküle können daher zum Identifizieren von Mikroorganismen eingesetzt werden, die sich zur Produktion von Feinchemikalien, bspw. durch Fermentationsverfahren, verwenden lassen. *C. glutamicum* selbst ist zwar nicht-pathogen, jedoch ist es mit anderen Corynebacterium-Arten, wie *Corynebacterium diphtheriae* (dem Erreger der Diphtherie) verwandt, die bedeutende Pathogene beim Menschen sind. Die Fähigkeit, das Vorhandensein von Corynebacterium-Arten zu identifizieren, kann daher auch eine signifikante klinische Bedeutung haben, z.B. bei diagnostischen Anwendungen. Diese Nukleinsäuremoleküle können zudem als Bezugspunkte zur Kartierung des *C. glutamicum-*Genoms oder von Genomen verwandter Organismen dienen.

Diese neuen Nukleinsäuremoleküle codieren Proteine, die als Glucose-6-Phosphat-Dehydrogenase bezeichnet werden.

Glucose-6-Phosphat-Dehydrogenase Gene aus Corynebakterien sind beispielsweise in EP 1108790A2 beschrieben. Allerdings codieren die dort beschriebenen Gene für Polypeptidsequenzen, die kürzer als die hier erfindungsgemäß beschriebenen sind. Die in EP 1108790A2 beschriebene Glucose-6-Phosphat-Dehydrogenase ist am N-Terminus um 30 Aminosäuren verkürzt gegenüber der hier beschriebenen Polypeptidsequenz.

Von Moritz et al. (Eur. J. Biochemistry 267, 3442-3452, 2000) wird die Isolierung einer Glucose-6-Phosphat-Dehydrogenase aus Corynebacterium glutamicum beschrieben. Die dort beschriebene N-Terminale Proteinsequenzierung ergibt ein Polypeptid, das mit einem Serin startet und verschieden von dem hier beschriebenenProtein ist.

Gegenstand der Erfindung sind neue Gene für Glucose-6-Phosphat-Dehydrogenase, die mit der Aminosäure in Position 1 , d.h. Formyl-Methionin , starten und an der Position 243 die Aminosäure Threonin codieren(Nummerierung bezogen auf SEQ ID NO:2).

Die erfindungsgemäßen Nukleinsäuremoleküle lassen sich zur genetischen Manipulation eines prokaryontischen Organismus verwenden, um ihn als Produzenten von einer oder mehreren Aminosäurebesser und effizienter zu machen. Die erfindungsgemäßen Moleküle lassen sich so modifizieren, daß die Ausbeute, Produktion und/oder die Effizienz der Produktion von einer oder mehreren Aminosäurenverbessert wird.

Die erfindungsgemäßen Moleküle können ferner an einem oder mehreren intrazellulären Signaltransduktionswegen beteiligt sein, die die Ausbeuten und/oder die Geschwindigkeit der Produktion einer oder mehrerer Aminosäurenvon *C. glutamicum* beeinflussen. Proteine, die bspw. für den Import von einem oder mehreren Zuckern aus dem extrazellulären Medium nötig sind (bspw. Hpr, Enzym I, oder ein Bestandteil des Enzym-II-Komplexes), werden bei Vorhandensein einer hinreichenden Menge Zucker in der Zelle häufig posttranslational modifiziert, so daß sie diesen Zucker nicht mehr importieren können. Die Zuckermenge, bei der das Transportsystem abgeschaltet wird, reicht zwar zur Aufrechterhaltung der normalen Zellfunktionen aus, jedoch schränkt sie die Überproduktion der gewünschten Aminosäureein. Es empfiehlt sich daher, die hier beschriebenen Proteine zu modifizieren, so daß sie auf eine solche negative Regulation nicht mehr ansprechen. Dadurch lassen sich höhere intrazelluläre Konzentrationen eines oder mehrerer Zucker und durch Extension eine effizientere Produktion oder höhere Ausbeuten von einer oder mehreren Aminosäurenaus prokaryontischenOrganismen erzielen, die diese mutanten Proteine enthalten.

Als Anhang A werden im folgenden die Nukleinsäuresequenzen des Sequenzprotokolls zusammen mit den in Tabelle 1 beschriebenen Sequenzveränderungen an der jeweiligen Position definiert.

Als Anhang B werden im folgenden die Polypeptidsequenzen des Sequenzprotokolls zusammen mit den in Tabelle 1 beschriebenen Sequenzveränderungen an der jeweiligen Position definiert.

Ein weiterer Aspekt der Erfindung betrifft Vektoren, bspw. rekombinante Expressionsvektoren, die die erfindungsgemäßen Nukleinsäuremoleküle enthalten, und prokaryontische Wirtszellen, in die diese Vektoren eingebracht worden sind. Bei einer Ausführungsform wird zur Herstellung eines G6PD-Proteins eine prokaryontische Wirtszelle verwendet, die in einem geeigneten Medium gezüchtet wird. Das G6PD-Protein kann dann aus dem Medium oder der prokaryontischen Wirtszelle isoliert werden.

Ein weiterer Aspekt der Erfindung betrifft einen genetisch veränderten Mikroorganismus, bei dem ein G6PD-Gen eingebracht oder verändert woreden ist. Das Genom des Mikroorganismus ist bei einer Ausführungsform durch Einbringen mindestens eines erfindungsgemäßen Nukleinsäuremoleküls verändert worden, das die mutierte G6PD-Sequenz als Transgen codiert. Bei einer anderen Ausführungsform ist ein endogenes G6PD-Gen im Genom des Mikroorganismus durch homologe Rekombination mit einem veränderten G6PD-Gen verändert, z.B. funktionell disruptiert, worden. Der Mikroorganismus gehört bei einer bevorzugten Ausführungsform zur Gattung *Corynebacterium* oder *Brevibacterium*, wobei *Corynebacterium glutamicum* besonders bevorzugt ist. Der Mikroorganismus wird in einer bevorzugten Ausführungsform auch zur Herstellung einer gewünschten Verbindung, wie einer Aminosäure, besonders bevorzugt Lysin, verwendet.

Eine weitere bevorzugte Ausführungsform sind prokaryontische Wirtszellen, die mehr als eine der in Anhang A beschriebenen Nukleinsäuremoleküle besitzen. Solche prokaryontischen Wirtszellen lassen sich auf verschiedene dem Fachmann bekannte Wege herstellen. Beispielsweise können sie durch Vektoren, die mehrere der erfindungsgemäßen Nukleinsäuremoleküle tragen, transfiziert werden. Es ist aber auch möglich mit einem Vektor jeweils ein erfindungsgemäßes Nukleinsäuremolekül in die prokaryontische Wirtszelle einzubringen und deshalb mehrere Vektoren entweder gleichzeitig oder zeitlich abgestuft einzusetzen. Es können somit prokaryontische Wirtszellen konstruiert werden, die zahlreiche, bis zu mehreren Hundert der erfindungsgemäßen Nukleinsäuresequenzen tragen. Durch eine solche Akkumulation lassen sich häufig überadditive Effekte auf die prokaryontische Wirtszelle hinsichtlich der Aminosäuren-Produktivität erzielen.

Das isolierte G6PD-Protein oder sein Abschnitt kann am Import von energiereichen Kohlenstoff-Molekülen (bspw. Glucose, Fructose oder Saccharose) in *C*. *glutamicum* und zudem an einem oder mehreren intrazellulären Signaltransduktionswegen von *C*. *glutamicum* beteiligt sein.

Ein weiterer Aspekt der Erfindung betrifft ein Verfahren zur Herstellung einer AminosäureDas Verfahren sieht die Anzucht einer prokayontischen Zelle vor, die einen Vektor enthält, der die Expression eines erfindungsgemäßen Nukleinsäuremoleküls bewirkt, so daß eine Aminosäureproduziert wird. Dieses Verfahren umfaßt bei einer bevorzugten Ausführungsform zudem den Schritt der Gewinnung einer prokaryontischen Zelle, die einen solchen Vektor enthält, wobei die Zelle mit einem Vektor transfiziert ist, der die Expression einer Nukleinsäure bewirkt. Dieses Verfahren umfaßt bei einer weiteren bevorzugten Ausführungsform zudem den Schritt, bei dem die Aminosäureaus der Kultur gewonnen wird. Die Zelle gehört bei einer bevorzugten Ausführungsform zur Gattung *Corynebacterium* oder *Brevibacterium*.

### I. Feinchemikalien

Der Begriff "Feinchemikalie" ist im Fachgebiet bekannt und beinhaltet Moleküle, die von einem Organismus produziert werden und in verschiedenen Industriezweigen Anwendungen finden, wie bspw., jedoch nicht beschränkt auf die pharmazeutische Industrie, die Landwirtschafts-, und Kosmetik-Industrie. Diese Verbindungen umfassen organische Säuren, wie Weinsäure, Itaconsäure und Diaminopimelinsäure, sowohl proteinogene als auch nicht-proteinogene Aminosäuren, Purin- und Pyrimidinbasen, Nukleoside und Nukleotide (wie bspw. beschrieben in Kuninaka, A. (1996) Nucleotides and related compounds, S. 561-612, in Biotechnology Bd. 6, Rehm et al., Hrsg. VCH: Weinheim und den darin enthaltenen Zitaten), Lipide, gesättigte und ungesättigte Fettsäuren (bspw. Arachidonsäure), Diole (bspw. Propandiol und Butandiol), Kohlenhydrate (bspw. Hyaluronsäure und Trehalose), aromatische Verbindungen (bspw. aromatische Amine, Vanillin und Indigo), Vitamine und Cofaktoren (wie beschrieben in Ullmann's Encyclopedia of Industrial Chemistry, Bd. A27, "Vitamins", S. 443-613 (1996) VCH: Weinheim und den darin enthaltenen Zitaten; und Ong, A.S., Niki, E. und Packer, L. (1995) "Nutrition, Lipids, Health and Disease" Proceedings of the UNESCO/Confederation of Scientific and Technological Associations in Malaysia and the Society for Free Radical Research - Asien, abgehalten am 1.-3. Sept. 1994 in Penang, Malaysia, AOCS Press (1995)), Enzyme und sämtliche anderen von Gutcho (1983) in Chemicals by Fermentation, Noyes Data Corporation, ISBN: 0818805086 und den darin angegebenen Literaturstellen, beschriebenen Chemikalien. Der Metabolismus und die Verwendungen bestimmter Feinchemikalien sind nachstehend weiter erläutert.

### A. Aminosäure-Metabolismus und Verwendungen

Die Aminosäuren umfassen die grundlegenden Struktureinheiten sämtlicher Proteine und sind somit für die normalen Zellfunktionen essentiell. Der Begriff "Aminosäure" ist im Fachgebiet bekannt. Die proteinogenen Aminosäuren, von denen es 20 Arten gibt, dienen als Struktureinheiten für Proteine, in denen sie über Peptidbindungen miteinander verknüpft sind, wohingegen die nicht-proteinogenen Aminosäuren (von denen Hunderte bekannt sind) gewöhnlich nicht in Proteinen vorkommen (siehe Ullmann's Encyclopedia of Industrial Chemistry, Bd. A2, S. 57-97 VCH: Weinheim (1985)). Die Aminosäuren können in der D- oder L-Konfiguration vorliegen, obwohl L-Aminosäuren gewöhnlich der einzige Typ sind, den man in natürlich vorkommenden Proteinen vorfindet. Biosynthese- und Abbauwege von jeder der 20 proteinogenen Aminosäuren sind sowohl bei prokaryotischen als auch eukaryotischen Zellen gut charakterisiert (siehe bspw. Stryer, L. Biochemistry, 3. Auflage, S. 578-590 (1988)). Die "essentiellen" Aminosäuren (Histidin, Isoleucin, Leucin, Lysin, Methionin, Phenylalanin, Threonin, Tryptophan und Valin), so bezeichnet, da sie aufgrund der Komplexität ihrer Biosynthese mit der Ernährung aufgenommen werden müssen, werden durch einfache Biosyntheseswege in die übrigen 11 "nichtessentiellen" Aminosäuren (Alanin, Arginin, Asparagin, Aspartat, Cystein, Glutamat, Glutamin, Glycin, Prolin, Serin und Tyrosin) umgewandelt. Höhere Tiere besitzen die Fähigkeit, einige dieser Aminosäuren zu synthetisieren, jedoch müssen die essentiellen Aminosäuren mit der Nahrung aufgenommen werden, damit eine normale Proteinsynthese stattfindet.

Abgesehen von ihrer Funktion bei der Proteinbiosynthese sind diese Aminosäuren interessante Chemikalien an sich, und man hat entdeckt, daß viele bei verschiedenen Anwendungen in der Nahrungsmittel-, Futter-, Chemie-, Kosmetik-, Landwirtschafts- und pharmazeutischen Industrie zum Einsatz kommen. Lysin ist nicht nur für die Ernährung des Menschen eine wichtige Aminosäure, sondern auch für monogastrische Tiere, wie Geflügel und Schweine. Glutamat wird am häufigsten als Geschmacksadditiv (Mononatriumglutamat, MSG) sowie weithin in der Nahrungsmittelindustrie verwendet, wie auch Aspartat, Phenylalanin, Glycin und Cystein. Glycin, L-Methionin und Tryptophan werden sämtlich in der pharmazeutischen Industrie verwendet. Glutamin, Valin, Leucin, Isoleucin, Histidin, Arginin, Prolin, Serin und Alanin werden in der pharmazeutischen Industrie und der Kosmetikindustrie verwendet. Threonin, Tryptophan und D-/L-Methionin sind weitverbreitete Futtermittelzusätze (Leuchtenberger, W. (1996) Amino acids - technical production and use, S. 466-502 in Rehm et al., (Hrsg.) Biotechnology Bd. 6, Kapitel 14a, VCH: Weinheim). Man hat entdeckt, daß sich diese Aminosäuren außerdem als Vorstufen für die Synthese von synthetischen Aminosäuren und Proteinen, wie N-Acetylcystein, S-Carboxymethyl-L-cystein, (S)-5-Hydroxytryptophan und anderen, in Ullmann's Encyclopedia of Industrial Chemistry, Bd. A2, S. 57-97, VCH, Weinheim, 1985 beschriebenen Substanzen eignen.

Die Biosynthese dieser natürlichen Aminosäuren in Organismen, die sie produzieren können, bspw. Bakterien, ist gut charakterisiert worden (für einen Überblick der bakteriellen Aminosäure-Biosynthese und ihrer Regulation, s. Umbarger, H.E. (1978) Ann. Rev. Biochem. 47: 533 - 606). Glutamat wird durch reduktive Aminierung von α-Ketoglutarat, einem Zwischenprodukt im Citronensäure-Zyklus, synthetisiert. Glutamin, Prolin und Arginin werden jeweils nacheinander aus Glutamat erzeugt. Die Biosynthese von Serin erfolgt in einem Dreischritt-Verfahren und beginnt mit 3-Phosphoglycerat (einem Zwischenprodukt bei der Glykolyse), und ergibt nach Oxidations-, Transaminierungs- und Hydrolyseschritten diese Aminosäure. Cystein und Glycin werden jeweils aus Serin produziert, und zwar die erstere durch Kondensation von Homocystein mit Serin, und die letztere durch Übertragung des Seitenketten-β-Kohlenstoffatoms auf Tetrahydrofolat, in einer durch Serintranshydroxymethylase katalysierten Reaktion. Phenylalanin und Tyrosin werden aus den Vorstufen des Glycolyse- und Pentosephosphatweges, Erythrose-4-phosphat und Phosphoenolpyruvat in einem 9-Schritt-Biosyntheseweg synthetisiert, der sich nur in den letzten beiden Schritten nach der Synthese von Prephenat unterscheidet. Tryptophan wird ebenfalls aus diesen beiden Ausgangsmolekülen produziert, jedoch erfolgt dessen Synthese in einem 11-Schritt-Weg. Tyrosin läßt sich in einer durch Phenylalaninhydroxylase katalysierten Reaktion auch aus Phenylalanin herstellen. Alanin, Valin und Leucin sind jeweils Biosyntheseprodukte aus Pyruvat, dem Endprodukt der Glykolyse. Aspartat wird aus Oxalacetat, einem Zwischenprodukt des Citratzyklus, gebildet. Asparagin, Methionin, Threonin und Lysin werden jeweils durch Umwandlung von Aspartat produziert. Isoleucin wird aus Threonin gebildet. In einem komplexen 9-Schritt-Weg erfolgt die Bildung von Histidin aus 5-Phosphoribosyl-1-pyrophosphat, einem aktivierten Zucker.

Aminosäuren, deren Menge den Proteinbiosynthesebedarf der Zelle übersteigt, können nicht gespeichert werden, und werden stattdessen abgebaut, so daß Zwischenprodukte für die Haupt-Stoffwechselwege der Zelle bereitgestellt werden (für einen Überblick siehe Stryer, L., Biochemistry, 3. Aufl. Kap. 21 "Amino Acid Degradation and the Urea Cycle"; S 495-516 (1988)). Die Zelle ist zwar in der Lage, ungewünschte Aminosäuren in nützliche Stoffwechsel-Zwischenprodukte umzuwandeln, jedoch ist die Aminosäureproduktion hinsichtlich der Energie, der Vorstufenmoleküle und der für ihre Synthese nötigen Enzyme aufwendig. Es überrascht daher nicht, daß die Aminosäure-Biosynthese durch Feedback-Hemmung reguliert wird, wobei das Vorliegen einer bestimmten Aminosäure ihre eigene Produktion verlangsamt oder ganz beendet (für einen Überblick über den Rückkopplungs-Mechanismus bei Aminosäure-Biosynthesewegen, siehe Stryer, L., Biochemistry, 3. Aufl., Kap. 24, "Biosynthesis of Amino Acids and Heme", S. 575-600 (1988)). Der Ausstoß einer bestimmten Aminosäure wird daher durch die Menge dieser Aminosäure in der Zelle eingeschränkt.

### B. Vitamine, Cofaktoren und Nutrazeutika-Metabolismus sowie Verwendungen

Vitamine, Cofaktoren und Nutrazeutika umfassen eine weitere Gruppe von Molekülen. Höhere Tiere haben die Fähigkeit verloren, diese zu synthetisieren und müssen sie somit aufnehmen, obwohl sie leicht durch andere Organismen, wie Bakterien, synthetisiert werden. Diese Moleküle sind entweder biologisch aktive Moleküle an sich oder Vorstufen von biologisch aktiven Substanzen, die als Elektronenträger oder Zwischenprodukte bei einer Reihe von Stoffwechselwegen dienen. Diese Verbindungen haben neben ihrem Nährwert auch einen signifikanten industriellen Wert als Farbstoffe, Antioxidantien und Katalysatoren oder andere Verarbeitungs-Hilfsstoffe. (Für einen Überblick über die Struktur, Aktivität und die industriellen Anwendungen dieser Verbindungen siehe bspw. Ullmann's Encyclopedia of Industrial Chemistry, "Vitamins", Bd. A27, S. 443-613, VCH: Weinheim, 1996). Der Begriff "Vitamin" ist im Fachgebiet bekannt und umfaßt Nährstoffe, die von einem Organismus für eine normale Funktion benötigt werden, jedoch nicht von diesem Organismus selbst synthetisiert werden können. Die Gruppe der Vitamine kann Cofaktoren und nutrazeutische Verbindungen umfassen. Der Begriff "Cofaktor" umfaßt nicht-proteinartige Verbindungen, die für das Auftreten einer normalen Enzymaktivität nötig sind. Diese Verbindungen können organisch oder anorganisch sein; die erfindungsgernäßen Cofaktor-Moleküle sind vorzugsweise orga nisch. Der Begriff "Nutrazeutikum" umfaßt Nahrungsmittelzusätze, die bei Pflanzen und Tieren, insbesondere dem Menschen, gesundheitsfördernd sind. Beispiele solcher Moleküle sind Vitamine, Antioxidantien und ebenfalls bestimmte Lipide (z.B. mehrfach ungesättigte Fettsäuren).

Die Biosynthese dieser Moleküle in Organismen, die zu ihrer Produktion befähigt sind, wie Bakterien, ist umfassend charakterisiert worden (Ullmann's Encyclopedia of Industrial Chemistry, "Vitamins", Bd. A27, S. 443-613, VCH: Weinheim, 1996, Michal, G. (1999) Biochemical Pathways: An Atlas of Biochemistry and Molecular Biology, John Wiley & Sons; Ong, A.S., Niki, E. und Packer, L. (1995) "Nutrition, Lipids, Health and Disease" Proceedings of the UNESCO/Confederation of Scientific and Technological Associations in Malaysia and the Society for free Radical Research - Asien, abgehalten am 1.-3. Sept. 1994 in Penang, Malaysia, AOCS Press, Champaign, IL X, 374 S).

Thiamin (Vitamin B₁) wird durch chemisches Kuppeln von Pyrimidin und Thiazol-Einheiten gebildet. Riboflavin (Vitamin B₂) wird aus Guanosin-5'-triphosphat (GTP) und Ribose-5'-phosphat synthetisiert. Riboflavin wiederum wird zur Synthese von Flavinmononukleotid (FMN) und Flavinadenindinukleotid (FAD) eingesetzt. Die Familie von Verbindungen, die gemeinsam als "Vitamin B6" bezeichnet werden (bspw. Pyridoxin, Pyridoxamin, Pyridoxal-5'-phosphat und das kommerziell verwendete Pyridoxinhydrochlorid), sind alle Derivate der gemeinsamen Struktureinheit 5-Hydroxy-6-methylpyridin. Panthothenat (Pantothensäure, R-(+)-N-(2,4-Dihydroxy-3,3-dimethyl-1-oxobutyl)-β-alanin) kann entweder durch chemische Synthese oder durch Fermentation hergestellt werden. Die letzten Schritte bei der Pantothenat-Biosynthese bestehen aus der ATP-getriebenen Kondensation von β-Alanin und Pantoinsäure. Die für die Biosyntheseschritte für die Umwandlung in Pantoinsäure, in β-Alanin und zur Kondensation in Pantothensäure verantwortlichen Enzyme sind bekannt. Die metabolisch aktive Form von Pantothenat ist Coenzym A, dessen Biosynthese über 5 enzymatische Schritte verläuft. Pantothenat, Pyridoxal-5'-phosphat, Cystein und ATP sind die Vorstufen von Coenzym A. Diese Enzyme katalysieren nicht nur die Bildung von Pantothenat, sondern auch die Produktion von (R)-Pantoinsäure, (R)-Pantolacton, (R)-Panthenol (Provitamin B₅), Pantethein (und seinen Derivaten) und Coenzym A.

Die Biosynthese von Biotin aus dem Vorstufenmolekül Pimeloyl-CoA in Mikroorganismen ist ausführlich untersucht worden, und man hat mehrere der beteiligten Gene identifiziert. Es hat sich herausgestellt, daß viele der entsprechenden Proteine an der Fe-Cluster-Synthese beteiligt sind und zu der Klasse der nifS-Proteine gehören. Die Liponsäure wird von der Octanonsäure abgeleitet und dient als Coenzym beim Energie-Metabolismus, wo sie Bestandteil des Pyruvatdehydrogenasekomplexes und des α-Ketoglutaratdehydrogenasekomplexes wird. Die Folate sind eine Gruppe von Substanzen, die alle von der Folsäure abgeleitet werden, die wiederum von L-Glutaminsäure, p-Aminobenzoesäure und 6-Methylpterin hergeleitet ist. Die Biosynthese der Folsäure und ihrer Derivate, ausgehend von den metabolischen Stoffwechselzwischenprodukten Guanosin-5'-triphosphat (GTP), L-Glutaminsäure und p-Aminobenzoesäure ist in bestimmten Mikroorganismen eingehend untersucht worden.

Corrinoide (wie die Cobalamine und insbesondere Vitamin B₁₂) und die Porphyrine gehören zu einer Gruppe von Chemikalien, die sich durch ein Tetrapyrrol-Ringsystem auszeichnen. Die Biosynthese von Vitamin B₁₂ ist hinreichend komplex, daß sie noch nicht vollständig charakterisiert worden ist, jedoch ist inzwischen ein Großteil der beteiligten Enzyme und Substrate bekannt. Nikotinsäure (Nikotinat) und Nikotinamid sind Pyridin-Derivate, die auch als "Niacin" bezeichnet werden. Niacin ist die Vorstufe der wichtigen Coenzyme NAD (Nikotinamidadenindinukleotid) und NADP (Nikotinamidadenindinukleotidphosphat) und ihrer reduzierten Formen.

Die Produktion dieser Verbindungen im Großmaßstab beruht größtenteils auf zellfreien chemischen Synthesen, obwohl einige dieser Chemikalien ebenfalls durch großangelegte Anzucht von Mikroorganismen produziert worden sind, wie Riboflavin, Vitamin B₆, Pantothenat und Biotin. Nur Vitamin B₁₂ wird aufgrund der Komplexität seiner Synthese lediglich durch Fermentation produziert. In-vitro-Verfahren erfordern einen erheblichen Aufwand an Materialien und Zeit und häufig an hohen Kosten.

### C. Purin-, Pyrimidin-, Nukleosid- und Nukleotid-Metabolismus und Verwendungen

Gene für den Purin- und Pyrimidin-Stoffwechsel und ihre entsprechenden Proteine sind wichtige Ziele für die Therapie von Tumorerkrankungen und Virusinfektionen. Der Begriff "Purin" oder "Pyrimidin" umfaßt stickstoffhaltige Basen, die Bestandteil der Nukleinsäuren, Coenzyme und Nukleotide sind. Der Begriff "Nukleotid" beinhaltet die grundlegenden Struktureinheiten der Nukleinsäuremoleküle, die eine stickstoffhaltige Base, einen Pentose-Zucker (bei RNA ist der Zucker Ribose, bei DNA ist der Zucker D-Desoxyribose) und Phosphorsäure umfassen. Der Begriff "Nukleosid" umfaßt Moleküle, die als Vorstufen von Nukleotiden dienen, die aber im Gegensatz zu den Nukleotiden keine Phosphorsäureeinheit aufweisen. Durch Hemmen der Biosynthese dieser Moleküle oder ihrer Mobilisation zur Bildung von Nukleinsäuremolekülen ist es möglich, die RNA- und DNA-Synthese zu hemmen; wird diese Aktivität zielgerichtet bei kanzerogenen Zellen gehemmt, läßt sich die Teilungs- und Replikations-Fähigkeit von Tumorzellen hemmen.

Es gibt zudem Nukleotide, die keine Nukleinsäuremoleküle bilden, jedoch als Energiespeicher (d.h. AMP) oder als Coenzyme (d.h. FAD und NAD) dienen.

Mehrere Veröffentlichungen haben die Verwendung dieser Chemikalin für diese medizinischen Indikationen beschrieben, wobei der Purin- und/oder Pyrimidin-Metabolismus beeinflußt wird (bspw. Christopherson, R.I. und Lyons, S.D. (1990) "Potent inhibitors of de novo pyrimidine and purine biosynthesis as chemotherapeutic agents", Med. Res. Reviews 10: 505-548). Untersuchungen an Enzymen, die am Purin- und Pyrimidin-Metabolismus beteiligt sind, haben sich auf die Entwicklung neuer Medikamente konzentriert, die bspw. als Immunsuppressionsmittel oder Antiproliferantien verwendet werden können (Smith, J.L. "Enzymes in Nucleotide Synthesis" Curr. Opin. Struct. Biol. 5 (1995) 752-757; Biochem. Soc. Transact. 23 (1995) 877-902). Die Purin- und Pyrimidinbasen, Nukleoside und Nukleotide haben jedoch auch andere Einsatzmöglichkeiten: als Zwischenprodukte bei der Biosysnthese verschiedener Feinchemikalien (z.B. Thiamin, S-Adenosyl-methionin, Folate oder Riboflavin), als Energieträger für die Zelle (bspw. ATP oder GTP) und für Chemikalien selbst, werden gewöhnlich als Geschmacksverstärker verwendet (bspw. IMP oder GMP) oder für viele medizinische Anwendungen (siehe bspw. Kuninaka, A., (1996) "Nucleotides and Related Compounds in Biotechnology Bd. 6, Rehm et al., Hrsg. VCH: Weinheim, S. 561-612). Enzyme, die am Purin-, Pyrimidin-, Nukleosid- oder Nukleotid-Metabolismus beteiligt sind, dienen auch immer stärker als Ziele, gegen die Chemikalien für den Pflanzenschutz, einschließlich Fungiziden, Herbiziden und Insektiziden entwickelt werden.

Der Metabolismus dieser Verbindungen in Bakterien ist charakterisiert worden (für Übersichten siehe bspw. Zalkin, H. und Dixon, J.E. (1992) "De novo purin nucleotide biosynthesis" in Progress in Nucleic Acids Research and Molecular biology, Bd. 42, Academic Press, S. 259-287; und Michal, G. (1999) "Nucleotides and Nucleosides"; Kap. 8 in : Biochemical Pathways: An Atlas of Biochemistry and Molecular Biology, Wiley, New York). Der Purin-Metabolismus, das Objekt intesiver Forschung, ist für das normale Funktionieren der Zelle essentiell. Ein gestörter Purin-Metabolismus in höheren Tieren kann schwere Erkrankungen verursachen, bspw. Gicht. Die Purinnukleotide werden über eine Reihe von Schritten über die Zwischenverbindung Inosin-5'-phosphat (IMP) aus Ribose-5-phosphat synthetisiert, was zur Produktion von Guanosin-5'-monophosphat (GMP) oder Adenosin-5'-monophosphat (AMP) führt, aus denen sich die als Nukleotide verwendeten Triphosphatformen leicht herstellen lassen. Diese Verbindungen werden auch als Energiespeicher verwendet, so daß ihr Abbau Energie für viele verschiedene biochemische Prozesse in der Zelle liefert. Die Pyrimidinbiosynthese erfolgt über die Bildung von Uridin-5'-monophosphat (UMP) aus Ribose-5-phosphat. UMP wiederum wird in Cytidin-5'-triphosphat (CTP) umgewandelt. Die Desoxyformen sämtlicher Nukleotide werden in einer Einschritt-Reduktionsreaktion aus der Diphosphat-Riboseform des Nukleotides zur Diphosphat-Desoxyriboseform des Nukleotides hergestellt. Nach der Phosphorylierung können diese Moleküle an der DNA-Synthese teilnehmen.

### D. Trehalose-Metabolismus und Verwendungen

Trehalose besteht aus zwei Glucosemolekülen, die über α,α-1,1-Bindung miteinander verknüpft sind. Sie wird gewöhnlich in der Nahrungsmittelindustrie als Süßstoff, als Additiv für getrocknete oder gefrorene Nahrungsmittel sowie in Getränken verwendet. Sie wird jedoch auch in der pharmazeutischen Industrie, der Kosmetik- und Biotechnologie-Industrie angewendet (s. bspw. Nishimoto et al., (1998) US-Patent Nr. 5 759 610; Singer, M.A. und Lindquist, S. Trends Biotech. 16 (1998) 460-467; Paiva, C.L.A. und Panek, A.D. Biotech Ann. Rev. 2 (1996) 293-314; und Shiosaka, M.J. Japan 172 (1997) 97-102). Trehalose wird durch Enzyme von vielen Mikroorganismen produziert und auf natürliche Weise in das umgebende Medium abgegeben, aus dem sie durch im Fachgebiet bekannte Verfahren gewonnen werden kann.

### II. Erfindungsgemäße Elemente und Verfahren

Die vorliegende Erfindung beruht zumindest teilweise auf der Entdeckung von neuen Molekülen, die hier als G6PD-Nukleinsäure- und -Protein-Moleküle bezeichnet werden und an der Aufnahme energiereicher Kohlenstoffmoleküle (z.B. Glucose, Saccharose und Fructose) in *C*. *glutamicum* beteiligt sind und auch an einem oder mehreren intrazellulären Signaltransduktionswegen in diesen Mikroorganismen beteiligt sein können. Bei einer Ausführungsform importieren die G6PD-Moleküle energiereiche Kohlenstoffmoleküle in die Zelle, wo die durch ihren Abbau erzeugte Energie zum Antreiben energetisch weniger begünstigter biochemischer Reaktionen eingesetzt wird. Ihre Abbauprodukte können als Zwischenprodukte oder Vorstufen für eine Reihe anderer Stoffwechselwege eingesetzt werden. Bei einer anderen Ausführungsform können die G6PD-Moleküle an einem oder mehreren intrazellulären Signaltransduktionswegen teilnehmen, wobei die Gegenwart einer modifizierten Form eines G6PD-Moleküls (z.B. ein phosphoryliertes G6PD-Protein) an einer Signaltransduktionskaskade teilnehmen kann, die einen oder mehrere Zellvorgänge reguliert. Die Aktivität der erfindungsgemäßen G6PD-Moleküle hat bei einer bevorzugten Ausführungsform eine Auswirkung auf die Produktion einer gewünschten Aminosäuredurch diesen prokaryontischen Organismus. Bei einer besonders bevorzugten Ausführungsform haben die erfindungsgemäßen G6PD-Moleküle eine modulierte Aktivität, so daß die Ausbeute, Produktion oder Effizienz der Produktion von einer oder mehreren Aminosäuren aus C. glutamicum ebenfalls moduliert sind.

Die erfindungsgemäßen G6PD-Moleküle sind in einer anderen Ausführungsform befähigt, die Produktion eines gewünschten Moleküls, wie einer Aminosäure, in einem Mikroorganismus, wie *C*. *glutamicum,* zu modulieren. Mit Hilfe von Genrekombinationstechniken lassen sich ein oder mehrere erfindungsgemäße G6PD-Proteine derart manipulieren, daß ihre Funktion moduliert ist. Bspw. kann ein Protein, das am G6PD-vermittelten Import von Glucose beteiligt ist, so verändert werden, daß es optimale Aktivität aufweist, und das G6PD-System für den Import von Glucose kann somit grö-ßere Mengen Glucose in die Zelle beförden. Glucosemoleküle werden nicht nur als Energiequelle für energetisch ungünstige biochemische Reaktionen, wie die Biosynthese von Aminosäuren verwendet, sondern auch als Vorstufen und Zwischenprodukte bei einer Reihe von Aminosäuren-Biosynthesewegen (bspw. wird Serin aus 3-Phosphoglycerat synthetisiert). In jedem Fall läßt sich die Gesamtausbeute oder die Produktionsrate einer dieser gewünschten Aminosäurenerhöhen, und zwar durch Erhöhen der Energie, die verfügbar ist, damit diese Produktion stattfindet, oder durch Vergrößern der Verfügbarkeit der Verbindungen, die nötig sind, damit diese Produktion stattfindet.

Als Ausgangspunkt zur Herstellung der erfindungsgemäßen Nukleinsäuresequenzen eignet sich das Genom eines *Corynebacterium glutamicum*-Stammes, der von der American Type Culture Collection unter der Bezeichnung ATCC 13032 erhältlich ist.

Von diesen Nukleinsäuresequenzen lassen sich durch die in Tabelle 1 bezeichneten Veränderungen die erfindungsgemäßen Nukleinsäuresequenzen mit üblichen Verfahren herstellen.

Das G6PD-Protein kann am Transport von energiereichen kohlenstoffhaltigen Molekülen, wie Glucose, in *C. glutamicum* oder an einer intrazellulären Signaltransduktion in diesem Mikroorganismus beteiligt sein, oder können eine oder mehrere der in Tabelle 1 aufgeführten Aktivitäten aufweisen.

In den nachstehenden Unterabschnitten sind verschiedene Aspekte der Erfindung ausführlicher beschrieben:

### A. Isolierte Nukleinsäuremoleküle

Ein Aspekt der Erfindung betrifft isolierte Nukleinsäuremoleküle, die G6PD-Polypeptide codieren. Der Begriff "Nukleinsäuremolekül" soll DNA-Moleküle (z.B. cDNA oder genomische DNA) und RNA-Moleküle (z.B. mRNA) sowie DNA- oder RNA-Analoga, die mittels Nukleotidanaloga erzeugt werden, umfassen. Dieser Begriff umfaßt zudem die am 3'- und am 5'-Ende des codierenden Genbereichs gelegene untranslatierte Sequenz: mindestens etwa 100 Nukleotide der Sequenz stromaufwärts des 5'-Endes des codierenden Bereichs und mindestens etwa 20 Nukleotide der Sequenz stromabwärts des 3'-Endes des codierenden Genbereichs. Das Nukleinsäuremolekül kann einzelsträngig oder doppelsträngig sein, ist aber vorzugsweise eine doppelsträngige DNA. Ein "isoliertes" Nukleinsäuremolekül wird aus anderen Nukleinsäuremolekülen abgetrennt, die in der natürlichen Quelle der Nukleinsäure zugegen sind. Eine "isolierte" Nukleinsäure hat vorzugsweise keine Sequenzen, die die Nukleinsäure in der genomischen DNA des Organismus, aus dem die Nukleinsäure stammt, natürlicherweise flankieren (bspw. Sequenzen, die sich am 5'- bzw. 3'-Ende der Nukleinsäure befinden). In verschiedenen Ausführungsformen kann bspw. das isolierte G6PD-Nukleinsäuremolekül weniger als etwa 5 kb, 4 kb, 3 kb, 2 kb, 1 kb, 0,5 kb oder 0,1 kb der Nukleotidsequenzen, die natürlicherweise das Nukleinsäuremolekül in der genomischen DNA der Zelle, aus der die Nukleinsäure stammt (bspw. eine *C*. *glutamicum-*Zelle) flankieren. Ein "isoliertes" Nukleinsäuremolekül, wie ein cDNA-Molekül kann überdies im wesentlichen frei von einem anderen zellulären Material oder Kulturmedium sein, wenn es durch rekombinante Techniken hergestellt wird, oder frei von chemischen Vorstufen oder anderen Chemikalien sein, wenn es chemisch synthetisiert wird.

Ein erfindungsgemäßes Nukleinsäuremolekül, bspw. ein Nukleinsäuremolekül mit einer Nukleotidsequenz aus Anhang A, kann mittels molekularbiologischer Standard-Techniken und der hier bereitgestellten Sequenzinformation hergestellt werden. Bspw. kann eine *C*. *glutamicum*-G6PD-cDNA aus einer *C*. *glutamicum*-Bank isoliert werden, indem eine vollständige Sequenz aus Anhang A oder ein Abschnitt davon als Hybridisierungssonde und Standard-Hybridisierungstechniken (wie bspw. beschrieben in Sambrook, J., Fritsch, E.F. und Maniatis, T. Molecular Cloning: A Laboratory Manual. 2. Aufl. Cold Spring Harbor Laboratory, Cold Spring Harbor Laboratory Press, Cold Spring Harbor, NY, 1989) verwendet werden. Überdies läßt sich ein Nukleinsäuremolekül, umfassend eine vollständige Sequenz aus Anhang A oder ein Abschnitt davon, durch Polymerasekettenreaktion isolieren, wobei die Oligonukleotidprimer, die auf der Basis dieser Sequenz erstellt wurden, verwendet werden (z.B. kann ein Nukleinsäuremolekül, umfassend eine vollständige Sequenz aus Anhang A oder einen Abschnitt davon, durch Polymerasekettenreaktion isoliert werden, indem Oligonukleotidprimer verwendet werden, die auf der Basis dieser gleichen Sequenz aus Anhang A erstellt worden sind). Bspw. läßt sich mRNA aus normalen Endothelzellen isolieren (bspw. durch das Guanidiniumthiocyanat-Extraktionsverfahren von Chirgwin et al. (1979) Biochemistry 18: 5294-5299) und die cDNA kann mittels reverser Transkriptase (bspw. Moloney-MLV-Reverse-Transkriptase, erhältlich bei Gibco/BRL, Bethesda, MD, oder AMV-Reverse-Transkriptase, erhältlich von Seikagaku America, Inc., St. Petersburg, FL) hergestellt werden. Synthetische Oligonukleotidprimer für die Amplifizierung via Polymerasekettereaktion lassen sich auf der Basis einer der in Anhang A gezeigten Nukleotidsequenzen erstellen. Eine erfindungsgemäße Nukleinsäure kann mittels cDNA oder alternativ genomischer DNA als Matrize und geeigneten Oligonukleotidprimern gemäß PCR-Standard-Amplifikationstechniken amplifiziert werden. Die so amplifizierte Nukleinsäure kann in einen geeigneten Vektor kloniert werden und durch DNA-Sequenzanalyse charakterisiert werden. Oligonukleotide, die einer G6PD-Nukleotidsequenz entsprechen, können durch Standard-Syntheseverfahren, bspw. mit einem automatischen DNA-Synthesegerät, hergestellt werden.

Bei einer bevorzugten Ausführungsform umfaßt ein erfindungsgemäßes isoliertes Nukleinsäuremolekül eine der in Anhang A aufgeführten Nukleotidsequenzen.

Zusätlich zu natürlich vorkommenden Varianten der G6PD-Sequenz, die in der Population existieren können, ist der Fachmann sich ebenfalls dessen bewußt, daß Änderungen durch Mutation in eine Nukleotidsequenz von Anhang A eingebracht werden können, was zur Änderung der Aminosäuresequenz des codierten G6PD-Proteins führt, ohne daß die Funktionsfähigkeit des G6PD-Proteins beeinträchtigt wird. Bspw. lassen sich Nukleotidsusbtitutionen, die an "nicht-essentiellen" Aminosäureresten zu Aminosäuresubstitutionen führen, in einer Sequenz von Anhang A herstellen. Ein "nicht-essentieller" Aminosäurerest läßt sich in einer Wildtypsequenz von einem der G6PD-Proteine (Anhang B) verändern, ohne daß die Aktivität des G6PD-Proteins verändert wird, wohingegen ein "essentieller" Aminosäurerest für die G6PD-Proteinaktivität erforderlich ist. Andere Aminosäurereste jedoch (bspw. nichtkonservierte oder lediglich semikonservierte Aminosäurereste in der Domäne mit G6PD-Aktivität) können für die Aktivität nicht essentiell sein und lassen sich somit wahrscheinlich verändern, ohne daß die G6PD-Aktivität verändert wird.

Ein isoliertes Nukleinsäuremolekül, das ein G6PD-Protein codiert, das zu einer Proteinsequenz aus Anhang B homolog ist, kann durch Einbringen von einer oder mehreren Nukleotidsubstitutionen, -additionen oder -deletionen in eine Nukleotidsequenz aus Anhang A erzeugt werden, so daß eine oder mehrere Aminosäuresubstitutionen, - additionen oder -deletionen in das codierte Protein eingebracht werden. Die Mutationen können in eine der Sequenzen aus Anhang A durch Standard-Techniken eingebracht werden, wie stellengerichtete Mutagenese und PCR-vermittelte Mutagenese. Vorzugsweise werden konservative Aminosäuresubstitutionen an einer oder mehreren der vorhergesagten nicht-essentiellen Aminosäureresten eingeführt. Bei einer "konservativen Aminosäuresubstitution" wird der Aminosäurerest durch einen Aminosäurerest mit einer ähnlichen Seitenkette ausgetauscht. Im Fachgebiet sind Familien von Aminosäureresten mit ähnlichen Seitenketten definiert worden. Diese Familien umfassen Aminosäuren mit basischen Seitenketten (z.B. Lysin, Arginin, Histidin), sauren Seitenketten (z.B. Asparaginsäure, Glutaminsäure), ungeladenen polaren Seitenketten (z.B. Glycin, Asparagin, Glutamin, Serin, Threonin, Tyrosin, Cystein), nicht-polaren Seitenketten, (bspw. Alanin, Valin, Leucin, Isoleucin, Prolin, Phenylalanin, Methionin, Tryptophan), beta-verzweigten Seitenketten (z.B. Threonin, Valin, Isoleucin) und aromatischen Seitenketten (z.B. Tyrosin, Phenylalanin, Tryptophan, Histidin). Ein vorhergesagter nicht-essentieller Aminosäurerest in einem G6PD-Protein wird somit vorzugsweise durch einen anderen Aminosäurerest der gleichen Seitenkettenfamilie ausgetauscht. In einer weiteren Ausführungsform können die Mutationen alternativ zufallsgemäß über die gesamte oder einen Teil der G6PD-codierenden Sequenz eingebracht werden, bspw. durch Sättigungsmutagenese, und die resultierenden Mutanten können auf die hier beschriebene G6PD-Aktivität untersucht werden, um Mutanten zu identifizieren, die eine G6PD-Aktivität beibehalten. Nach der Mutagenese von einer der Sequenzen aus Anhang A kann das codierte Protein rekombinant exprimiert werden, und die Aktivität des Proteins kann bspw. mit den hier beschriebenen Tests (siehe Beispiel 8 des Beispielteils) bestimmt werden.

### B. Rekombinante Expressions vektoren und Wirtszellen

Ein weiterer Aspekt der Erfindung betrifft Vektoren, vorzugsweise Expressionsvektoren, die eine Nukleinsäure enthalten, die ein G6PD-Protein codieren. Wie hier verwendet betrifft der Begriff "Vektor" ein Nukleinsäuremolekül, das eine andere Nukleinsäure transportieren kann, an welche es gebunden ist. Ein Vektortyp ist ein "Plasmid", was für eine zirkuläre doppelsträngige DNA-Schleife steht, in die zusätzlichen DNA-Segmente ligiert werden können. Ein weiterer Vektortyp ist ein viraler Vektor, wobei zusätzliche DNA-Segmente in das virale Genom ligiert werden können. Bestimmte Vektoren können in einer Wirtszelle, in die sie eingebracht worden sind, autonom replizieren (bspw. Bakterienvektoren, mit bakteriellem Replikationsursprung und episomale Säugetiervektoren). Andere Vektoren (z.B. nicht-episomale Säugetiervektoren) werden in das Genom einer Wirtszelle beim Einbringen in die Wirtszelle integriert und dadurch zusammen mit dem Wirtsgenom repliziert. Zudem können bestimmte Vektoren die Expression von Genen, mit denen sie funktionsfähig verbunden sind, steuern. Diese Vektoren werden als "Expressionsvektoren" bezeichnet. Gewöhnlich haben die Expressionsvektoren, die bei DNA-Rekombinationstechniken verwendet werden, die Form von Plasmiden. In der vorliegenden Beschreibung können "Plasmid" und "Vektor" austauschbar verwendet werden, da das Plasmid die am häufigsten verwendete Vektorform ist. Die Erfindung soll diese anderen Expressionsvektorformen, wie virale Vektoren (bspw. replikationsdefiziente Retroviren, Adenoviren und adenoverwandte Viren), die ähnliche Funktionen ausüben, umfassen.

Der erfindungsgemäße rekombinante Expressionsvektor umfaßt eine erfindungsgemäβe Nukleinsäure in einer Form, die sich zur Expression der Nukleinsäure in einer prokaryontischen Wirtszelle eignet, was bedeutet, daß die rekombinanten Expressionsvektoren eine oder mehrere regulatorische Sequenzen, ausgewählt auf der Basis der zur Expression zu verwendenden Wirtszellen, die mit der zu exprimierenden Nukleinsäuresequenz funktionsfähig verbunden ist, umfaßt. In einem rekombinanten Exprtessionsvektor bedeutet "funktionsfähig verbunden", daß die Nukleotidsequenz von Interesse derart an die regulatorische(n) Sequenz(en) gebunden ist, daß die Expression der Nukleotidsequenz möglich ist (bspw. in einem In-vitro-Transkriptions-/Translationssystem oder in einer Wirtszelle, wenn der Vektor in die prokaryontische-Wirtszelle eingebracht ist). Der Begriff "regulatorische Sequenz" soll Promotoren, Enhancer und andere Expressionskontrollelemente (bspw. Polyadenylierungssignale) umfassen. Diese regulatorischen Sequenzen sind bspw beschrieben in Goeddel: Gene Expression Technology: Methods in Enzymology 185, Academic Press, San Diego, CA (1990). Regulatorische Sequenzen umfassen solche, die die konstitutive Expression einer Nukleotidsequenz in vielen Wirtszelltypen steuern, und solche, die die direkte Expression der Nukleotidsequenz nur in bestimmten Wirtszellen steuern. Der Fachmann ist sich dessen bewußt, daß die Gestaltung eines Expressionsvektors von Faktoren abhängen kann, wie der Wahl der zu transformierenden Wirtszelle, dem Ausmaß der Expression des gewünschten Proteins usw. Die erfindungsgemäßen Expressionsvektoren können in die prokaryontische Wirtszellen eingebracht werden, so daß dadurch Proteine oder Peptide, einschließlich Fusionsproteinen oderpeptiden, die von den Nukleinsäuren, wie hier beschrieben, codiert werden, hergestellt werden (bspw. G6PD-Proteine, mutante Formen von G6PD-Proteinen, Fusionsproteine, usw.).

Die erfindungsgemäßen rekombinanten Expressionsvektoren können zur Expression von G6PD-Proteinen in prokaryotischen Zellen ausgestaltet sein. Bspw. können G6PD-Gene in bakteriellen Zellen, wie *C. glutamicum,* exprimiert werden. Geeignete Wirtszellen werden weiter erörtert in Goeddel, Gene Expression Technology: Methods in Enzymology 185, Academic Press, San Diego, CA (1990). Der rekombinante Expressionsvektor kann alternativ, bspw. mit T7-Promotorregulatorischen Sequenzen und T7-Polymerase, in vitro transkribiert und translatiert werden.

Die Expression von Proteinen in Prokaryonten erfolgt meist mit Vektoren, die konstitutive oder induzierbare Promotoren enthalten, die die Expression von Fusions- oder Nicht-Fusionsproteinen steuern. Fusionsvektoren steuern eine Reihe von Aminosäuren zu einem darin codierten Protein, gewöhnlich am Aminoterminus des rekombinanten Proteins, bei. Diese Fusionsvektoren haben gewöhnlich drei Aufgaben: 1) die Verstärkung der Expression von rekombinantem Protein; 2) die Erhöhung der Löslichkeit des rekombinanten Proteins; und 3) die Unterstützung der Reinigung des rekombinanten Proteins durch Wirkung als Ligand bei der Affinitätsreinigung. Bei Fusions-Expressionsvektoren wird oft eine proteolytische Spaltstelle an der Verbindungsstelle der Fusionseinheit und des rekombinanten Proteins eingebracht, so daß die Trennung des rekombinanten Proteins von der Fusionseinheit nach der Reinigung des Fusionsproteins möglich ist. Diese Enzyme und ihre entsprechenden Erkennungssequenzen umfassen Faktor Xa, Thrombin und Enterokinase.

Übliche Fusionsexpressionsvektoren umfassen pGEX (Pharmacia Biotech Inc; Smith, D.B. und Johnson, K.S. (1988) Gene 67: 31-40), pMAL (New England Biolabs, Beverly, MA) und pRIT 5 (Pharmacia, Piscataway, NJ), bei denen Glutathion-S-Transferase (GST), Maltose E-bindendes Protein bzw. Protein A an das rekombinante Zielprotein fusioniert wird. Bei einer Ausführungsform ist die codierende Sequenz des G6PD-Proteins in einen pGEX-Expressionsvektor kloniert, so daß ein Vektor erzeugt wird, der ein Fusionsprotein codiert, umfassend vom N-Terminus zum C-Terminus, GST - Thrombin-Spaltstelle - X-Protein. Das Fusionsprotein kann durch Affinitätschromatographie mittels Glutathion-Agarose-Harz gereinigt werden. Das rekombinante G6PD-Protein, das nicht mit GST fusioniert ist, kann durch Spaltung des Fusionsproteins mit Thrombin gewonnen werden.

Beispiele geeigneter induzierbarer Nicht-Fusions-Expressionsvektoren aus E. coli umfassen pTrc (Amann et al., (1988) Gene 69: 301 - 315) und pET 11d (Studier et al. Gene Expression Technology: Methods in Enzymology 185, Academic Press, San Diego, Kalifornien (1990) 60-89). Die Zielgenexpression aus dem pTrc-Vektor beruht auf der Transkription durch Wirts-RNA-Polymerase von einem Hybrid-trp-lac-Fusionspromotor. Die Zielgenexpression aus dem pET11 d-Vektor beruht auf der Transkription von einem T7-gn10-lac-Fusions-Promotor, die von einer coexprimierten viralen RNA-Polymerase (T7 gn1) vermittelt wird. Diese virale Polymerase wird von den Wirtsstämmen BL 21 (DE3) oder HMS174 (DE3) von einem residenten λ-Prophagen geliefert, der ein T7 gn1-Gen unter der Transkriptionskontrolle des lacUV 5-Promotors birgt.

Eine Strategie zur Maximierung der Expression des rekombinanten Proteins ist die Expression des Proteins in einem Wirtsbakterium, dessen Fähigkeit zur proteolytischen Spaltung des rekombinanten Proteins gestört ist (Gottesman, S. Gene Expression Technology: Methods in Enzymology 185, Academic Press, San Diego, Kalifornien (1990) 119-128). Eine weitere Strategie ist die Veränderung der Nukleinsäuresequenz der in einen Expressionsvektor zu inserierenden Nukleinsäure, so daß die einzelnen Codons für jede Aminosäure diejenigen sind, die vorzugsweise in einem zur Expression ausgewählten Bakterium, wie *C*. *glutamicum*, verwendet werden (Wada et al. (1992) Nucleic Acids Res. 20: 2111 - 2118). Diese Veränderung der erfindungsgemäßen Nukleinsäuresequenzen erfolgt durch Standard-DNA-Synthesetechniken.

Die Erfindung stellt zudem einen rekombinanten Expressionsvektor bereit, umfassend ein erfindungsgemäßes DNA Molekül, das in Antisense-Richtung in den Expressionsvektor kloniert ist. Dies bedeutet, daß das DNA-Molekül derart mit einer regulatorischen Sequenz funktionsfähig verbunden ist, daß die Expression (durch Transkription des DNA-.Moleküls) eines RNA-Moleküls, das zur G6PD-mRNA antisense ist, möglich ist. Es können regulatorische Sequenzen ausgewählt werden, die funktionsfähig an eine in Antisense-Richtung klonierte Nukleinsäure gebunden sind und die die kontinuierliche Expression des Antisense-RNA-Moleküls in einer Vielzahl von Zelltypen steuern, bspw. können virale Promotoren und/oder Enhancer oder regulatorische Sequenzen ausgewählt werden, die die konstitutive, gewebespezifische oder zelltypspezifische Expression von Antisense-RNA steuern. Der Antisense-Expressionsvektor kann in Form eines rekombinanten Plasmids, Phagemids oder attenuierten Virus vorliegen, in dem Antisense-Nukleinsäuren unter der Kontrolle eines hochwirksamen regulatorischen Bereichs produziert werden, dessen Aktivität durch den Zelltyp bestimmt wird, in den der Vektor eingebracht wird. Für eine Diskussion der Regulation der Genexpression mittels Antisense-Genen, siehe Weintraub, H. et al., Antisense-RNA as a molecular tool for genetic analysis, Reviews - Trends in Genetics, Bd. 1 (1) 1986.

Ein weiterer Aspekt der Erfindung betrifft die prokaryontischen Wirtszellen, in die ein erfindungsgemäßer rekombinanter Expressionsvektor eingebracht worden ist. Die Begriffe "Wirtszelle" und "rekombinante Wirtszelle" werden hier untereinander austauschbar verwendet. Es ist selbstverständlich, daß diese Begriffe nicht nur eine bestimmte Zielzelle, sondern auch die Nachkommen oder potentiellen Nachkommen dieser Zelle betreffen. Da in aufeinanderfolgenden Generationen aufgrund von Mutation oder Umwelteinflüssen bestimmte Modifikationen auftreten können, sind diese Nachkommen nicht unbedingt mit der Parentalzelle identisch, sind jedoch im Umfang des Begriffs, wie er hier verwendet wird, noch umfaßt.

Eine Wirtszelle kann eine prokaryotische Zelle sein. Bspw. kann ein G6PD-Protein in Bakterienzellen, wie *C. glutamicum*,) exprimiert werden. Andere geeignete prokaryontische Wirtszellen sind dem Fachmann geläufig. Mikroorganismen, die mit *Corynebacterium glutamicum* verwandt sind und sich geeignet als Wirtszellen für die erfindungsgemäßen Nukleinsäure- und Proteinmoleküle verwenden lassen, sind in Tabelle 3 aufgeführt.

Durch herkömmliche Transformations- oder Transfektionsverfahren läßt sich Vektor-DNA in prokaryotische Zellen einbringen. Die Begriffe "Transformation" und "Transfektion", wie sie hier verwendet werden, sollen eine Vielzahl von im Stand der Technik bekannten Verfahren zum Einbringen fremder Nukleinsäure (bspw. DNA) in eine Wirtszelle umfassen, einschließlich Calciumphosphat- oder Calciumchlorid-Copräzipitation, DEAE-Dextran-vermittelte Transfektion, Lipofektion oder Elektroporation. Geeignete Verfahren zur Transformation oder Transfektion von Wirtszellen lassen sich nachlesen in Sambrook et al. (Molecular Cloning: A Laboratory Manual. 2. Aufl. Cold Spring Harbor Laboratory, Cold Spring Harbor Laboratory Press, Cold Spring Harbor, NY, 1989) und anderen Labor-Handbüchern.

Zur Erzeugung eines homolog rekombinierten Mikroorganismus wird ein Vektor hergestellt, der zumindest einen Abschnitt eines G6PD-Gens enthält, in den eine Deletion, Addition oder Substitution eingebracht worden ist, um das G6PD-Gen zu verändern, bspw. funktionell zu disrumpieren. Dieses G6PD-Gen ist vorzugsweise ein *Corynebacterium glutamicum*-G6PD-Gen. Bei einer bevorzugten Ausführungsform ist der Vektor derart ausgestaltet, daß das endogene G6PD-Gen bei homologer Rekombination funktionell disrumpiert ist (d.h. nicht länger ein funktionelles Protein codiert, ebenfalls bezeichnet als "Knockout"-Vektor). Der Vektor kann alternativ derart ausgestaltet sein, daß das endogene G6PD-Gen bei homologer Rekombination mutiert oder anderweitig verändert ist, jedoch noch das funktionelle Protein codiert (z.B. kann der stromaufwärts gelegene regulatorische Bereich derart verändert sein, daß dadurch die Expression des endogenen G6PD-Proteins verändert wird.). Der veränderte Abschnitt des G6PD-Gens ist im homologen Rekombinationsvektor an seinem 5'- und 3'-Ende von zusätzlicher Nukleinsäure des G6PD-Gens flankiert, die eine homologe Rekombination zwischen dem exogenen G6PD-Gen, das von dem Vektor getragen wird, und einem endogenen G6PD-Gen in einem Mikroorganismus, ermöglicht. Die zusätzliche flankierende G6PD-Nukleinsäure ist für eine erfolgreiche homologe Rekombination mit dem endogenen Gen hinreichend lang. Gewöhnlich enthält der Vektor mehrere Kilobasen flankierende DNA (sowohl am 5'- als auch am 3'-Ende) (siehe z.B. Thomas, K.R. und Capecchi, M.R. (1987) Cell 51: 503 für eine Beschreibung von homologen Rekombinationsvektoren). Der Vektor wird in einen Mikroorganismus (z.B. durch Elektroporation) eingebracht, und Zellen, in denen das eingebrachte G6PD-Gen mit dem endogenen G6PD-Gen homolog rekombiniert ist, werden unter Verwendung im Fachgebiet bekannter Verfahren selektiert.

Bei einer anderen Ausführungsform können rekombinante Mikroorganismen produziert werden, die ausgewählte Systeme enthalten, die eine regulierte Expression des eingebrachten Gens ermöglichen. Der Einschluß eines G6PD-Gens in einem Vektor unter der Kontrolle des Lac-Operons ermöglicht z.B. die Expression des G6PD-Gens nur in Gegenwart von IPTG. Diese regulatorischen Systeme sind im Fachgebiet bekannt.

Eine erfindungsgemäße prokaryontische Wirtszelle, wie eine prokaryotische Wirtszelle in Kultur, kann zur Produktion (d.h. Expression) eines G6PD-Proteins verwendet werden. Die Erfindung stellt zudem Verfahren zur Produktion von G6PD-Proteinen unter Verwendung der erfindungsgemäßen prokaryontischen Wirtszellen bereit. Bei einer Ausführungsform umfaßt das Verfahren die Anzucht der erfindungsgemäßen prokaryontischen Wirtszelle (in die ein rekombinanter Expressionsvektor, der ein G6PD-Protein codiert, eingebracht worden ist, oder in deren Genom ein Gen eingebracht worden ist, das ein Wildtyp- oder verändertes G6PD-Protein codiert) in einem geeigneten Medium, bis das G6PD-Protein produziert worden ist. Das Verfahren umfaßt in einer weiteren Ausführungsform das Isolieren der G6PD-Proteine aus dem Medium oder der prokaryontischen Wirtszelle.

### C. Erfindungsgemäße Verwendungen und Verfahren

Die hier beschriebenen Nukleinsäuremoleküle, Vektoren und prokaryontischen Wirtszellen können in einem oder mehreren nachstehenden Verfahren verwendet werden: Identifikation von *C*. glutamicum und verwandten Organismen, Kartierung von Genomen von Organismen, die mit *C*. *glutamicum* verwandt sind, Identifikation und Lokalisation von *C*. *glutamicum*-Sequenzen von Interesse, Evolutionsstudien, Bestimmung von G6PD-Proteinbereichen, die für die Funktion notwendig sind, Modulation der Aktivität eines G6PD-Proteins; Modulation der Aktivität eines G6PD-Wegs; und Modulation der zellulären Produktion einer gewünschten Verbindung, wie einerAminosäure. Die erfindungsgemäßen G6PD-Nukleinsäuremoleküle haben eine Vielzahl von Verwendungen. Sie können zunächst zur Identifikation eines Organismus als *Corynebacterium glutamicum oder* naher Verwandten davon verwendet werden. Sie können zudem zur Identifikation von *C*. *glutamicum* oder eines Verwandten davon in einer Mischpopulation von Mikroorganismen verwendet werden. Die Erfindung stellt die Nukleinsäuresequenzen einer Reihe von *C*. *glutamicum*-Genen bereit. Durch Sondieren der extrahierten genomischen DNA einer Kultur einer einheitlichen oder gemischten Population von Mikroorganismen unter stringenten Bedingungen mit einer Sonde, die einen Bereich eines *C*. *glutamicum-*Gens umfaßt, das für diesen Organismus einzigartig ist, kann man bestimmen, ob dieser Organismus zugegen ist. *Corynebacterium glutamicum* selbst ist zwar nicht pathogen, jedoch ist es mit pathogenen Arten, wie *Corynebakterium diptheriae,* verwandt. Der Nachweis eines solchen Organismus ist von signifikanter klinischer Bedeutung.

Die erfindungsgemäßen Nukleinsäure- und Proteinmoleküle können als Marker für spezifische Bereiche des Genoms dienen. Dies ist nicht nur beim Kartieren des Genoms, sondern auch für funktionelle Studien von *C*. *glutamicum*-Proteinen nützlich. Zur Identifikation des Genombereichs, an den ein bestimmtes *C*. *glutamicum-DNA-*bindendes Protein bindet, kann das *C*. *glutamicum*-Genom bspw. gespalten werden, und die Fragmente mit dem DNA-bindenden Protein inkubiert werden. Diejenigen, die das Protein binden, können zusätzlich mit den erfindungsgemäßen Nukleinsäuremolekülen, vorzugsweise mit leicht nachweisbaren Markierungen, sondiert werden; die Bindung eines solchen Nukleinsäuremoleküls an das Genomfragment ermöglicht die Lokalisation des Fragmentes auf der genomischen Karte von *C*. *glutamicum,* und wenn dies mehrmals mit unterschiedlichen Enzymen durchgeführt wird, erleichtert es eine rasche Bestimmung der Nukleinsäuresequenz, an die das Protein bindet. Die erfindungsgemäßen Nukleinsäuremoleküle können zudem hinreichend homolog zu den Sequenzen verwandter Arten sein, so daß diese Nukleinsäuremoleküle als Marker für die Konstruktion einer genomischen Karte in verwandten Bakterien, wie *Brevibacterium lactofermentum,* dienen können.

Die erfindungsgemäßen G6PD-Nukleinsäuremoleküle eignen sich ebenfalls für Evolutions- und Proteinstrukturuntersuchungen. Das Zuckeraufnahmesystem, an dem die erfindungsgemäßen Moleküle beteiligt sind, wird von vielen Bakterien ausgenutzt; durch Vergleich der Sequenzen der erfindungsgemäßen Nukleinsäuremoleküle mit solchen, die ähnliche Enzyme aus anderen Organismen codieren, kann der Evolutions-Verwandschaftsgrad der Organismen bestimmt werden. Entsprechend ermöglicht ein solcher Vergleich die Bestimmung, welche Sequenzbereiche konserviert sind und welche nicht, was bei der Bestimmung solcher Bereiche des Proteins hilfreich sein kann, die für die Enzymfunktion essentiell sind. Dieser Typ der Bestimmung ist für Proteintechnologie-Untersuchungen wertvoll und kann einen Hinweis darauf geben, welches Protein Mutagenese tolerieren kann, ohne die Funktion zu verlieren.

Die Manipulation der erfindungsgemäßen G6PD-Nukleinsäuremoleküle kann die Produktion von G6PD-Proteinen mit funktionellen Unterschieden zu den Wildtyp-G6PD-Proteinen bewirken. Diese Proteine können hinsichtlich ihrer Effizienz oder Aktivität verbessert werden, können in größerer Anzahl als gewöhnlich in der Zelle zugegen sein, oder können hinsichtlich ihrer Effizienz oder Aktivität geschwächt sein.

Die erfindungsgemäßen G6PD-Moleküle können derart modifiziert sein, daß die Ausbeute, Produktion und/oder Effizienz der Produktion von einer oder mehreren Aminosäuren verbessert ist. Durch derartige Modifikation eines an der Glucoseaufnahme beteiligten G6PD-Proteins, daß es optimale Aktivität aufweist, kann das Ausmaß der Glucoseaufnahme oder die Geschwindigkeit, mit der die Glucose in die Zelle befördert wird, vergrößert werden. Der Abbau von Glucose und anderen Zuckern innerhalb der Zelle stellt die Energie bereit, die zum Antrieb energetisch ungünstiger biochemischer Reaktionen, wie solchen, die an der Biosynthese von Aminosäuren beteiligt sind, verwendet werden kann. Dieser Abbau stellt ebenfalls Zwischen- und Vorstufenmoleküle für die Biosynthese bestimmterAminosäuren bereit. Durch Erhöhung der Menge an intrazellulären energiereichen Kohlenstoffmolekülen durch Modifikation der erfindungsgemäßen G6PD-Moleküle kann man daher die Energie, die zur Durchführung von Stoffwechselwegen, die zur Produktion von ein oder mehreren Aminosäuren notwendig sind, verfügbar ist, und auch die intrazellulären Pools von Metaboliten, die für eine solche Produktion notwendig sind, vergrößern. Umgekehrt kann man durch Senken des Imports eines Zuckers, dessen Abbauprodukte eine Verbindung umfassen, die nur in Stoffwechselwegen verwendet werden, die mit Stoffwechselwegen zur Produktion einer gewünschten Aminosäure um Enzyme, Cofaktoren oder Zwischenprodukte konkurrieren, diesen Stoffwechselweg herunterregulieren und somit vielleicht die Produktion durch den gewünschten Biosyntheseweg vergrößern.

Die erfindungsgemäßen G6PD-Moleküle können an einem oder mehreren intrazellulären Signaltransduktionswegen, die die Ausbeuten und/oder Produktionsrate von einer oder mehreren Aminosäuren aus *C*. *glutamicum* beeinflussen, beteiligt sein. Bspw. werden Proteine, die für den Import von einem oder mehreren Zuckern aus dem extrazellulären Medium notwendig sind, (z.B. HPr, Enzym 1 oder ein Baustein eines Enzym-II-Komplexes) bei Anwesenheit einer hinreichenden Menge des Zuckers in der Zelle häufig posttranslational modifiziert, so daß sie nicht länger zum Import dieses Zuckers befähigt sind. Ein Beispiel hierfür erfolgt in *E*. *coli*, wo hohe intrazelluläre Fructose-1,6-bisphosphatspiegel die Phosphorylierung von HPr an Serin-46 bewirken, woraufhin dieses Molekül nicht länger am Transport eines Zuckers teilnehmen kann. Dieser intrazelluläre Zuckerspiegel, bei dem das Transportsystem abgeschaltet wird, kann zwar hinreichend sein, um die normale Funktion der Zelle aufrechtzuerhalten, ist jedoch für die Überproduktion der gewünschten Aminosäure einschränkend. Es ist daher wünschenswert, die erfindungsgemäßen G6PD-Proteine zu modifizieren, so daß sie nicht länger für eine solche negative Regulation empfänglich sind. Dadurch werden größere intrazelluläre Konzentrationen von einem oder mehreren Zuckern, und durch Extension, eine effizientere Produktion oder größere Ausbeuten von einer oder mehreren Aminosäuren aus Organismen, die diese mutanten G6PD-Proteine enthalten, erzielt.

Diese vorstehend genannte Liste von Mutagenesestrategien für G6PD-Proteine, die erhöhte Ausbeuten einer gewünschten Verbindung bewirken sollen, soll nicht einschränkend sein; Variationen dieser Mutagenesestrategien sind dem Fachmann leicht ersichtlich. Durch diese Mechanismen können die erfindungsgemäßen Nukleinsäurmoleküle- verwendet werden, um *C. glutamicum* oder verwandte Bakterienstämme, die mutierte G6PD-Nukleinsäuremoleküle exprimieren, zu erzeugen, so daß die Ausbeute, Produktion und/oder Effizienz der Produktion einer gewünschten Verbindung verbessert wird. Die gewünschte Verbindung kann ein natürliches Produkt von *C*. *glutamicum* sein, welches die Endprodukte der Biosynthesewege und Zwischenprodukte natürlich vorkommender metabolischer Wege sowie Moleküle umfaßt, die im Metabolismus von *C*. *glutamicum* nicht natürlich vorkommen, die jedoch von einem erfindungsgemäßen *C*. *glutamicum-*Stamm produziert werden.

Diese Erfindung wird durch die nachstehenden Beispiele weiter veranschaulicht, die nicht als einschränkend aufgefaßt werden sollen. Beispiele

### Beispiel 1: Präparation der gesamten genomischen DNA aus Corynebacterium glutamicum ATCC13032

Eine Kultur von *Corynebacterium glutamicum* (ATCC 13032) wurde über Nacht bei 30_C unter starkem Schütteln in BHI-Medium (Difco) gezüchtet. Die Zellen wurden durch Zentrifugation geerntet, der Überstand wurde verworfen, und die Zellen wurden in 5ml Puffer I (5% des Ursprungsvolumens der Kultur- sämtliche angegebenen Volumina sind für 100 ml Kulturvolumen berechnet) resuspendiert. Die Zusammensetzung von Puffer I: 140,34 g/l Saccharose, 2,46 g/l MgSO₄ - 7 H₂O, 10 ml/l KH₂PO₄-Lösung (100g/l, mit KOH eingestellt auf pH-Wert 6,7), 50 ml/l M12-Konzentrat (10 g/l (NH₄)₂SO₄, 1 g/l NaCl, 2 g/l MgSO₄·7 H₂O, 0,2 g/l CaCl₂, 0,5 g/l Hefe-Extrakt (Difco), 10 ml/l Spurenelemente-Mischung (200 mg/l FeSO₄·H₂O, 10 mg/l ZnSO₄·7 H₂O, 3 mg/l MnCl₂·4 H₂O, 30 mg/l H₃BO₃, 20 mg/l CoCl₂·6 H₂O, 1 mg/l NiCl₂·6 H₂O, 3 mg/l Na₂MoO₄·2 H₂O, 500 mg/l Komplexbildner (EDTA oder Citronensäure), 100 ml/l Vitamingemisch (0,2 ml/l Biotin, 0,2 mg/l Folsäure, 20 mg/l p-Aminobenzoesäure, 20 mg/l Riboflavin, 40 mg/l Ca-Panthothenat, 140 mg/l Nikotinsäure, 40 mg/l Pyridoxolhydrochlorid, 200 mg/l Myoinositol). Lysozym wurde in einer Endkonzentration von 2,5 mg/ml zur Suspension gegeben. Nach etwa 4 Std. Inkubation bei 37°C wurde die Zellwand abgebaut, und die erhaltenen Protoplasten wurden durch Zentrifugation geerntet. Das Pellet wurde einmal mit 5 ml Puffer 1 und einmal mit 5 ml TE-Puffer (10 mM Tris-HCl, 1 mM EDTA, pH-Wert 8) gewaschen. Das Pellet wurde in 4 ml TE-Puffer resuspendiert, und 0,5 ml SDS-Lösung (10%) und 0,5 ml NaCI-Lösung (5 M) wurden zugegeben. Nach Zugabe von Proteinase K in einer Endkonzentration von 200 µg/ml wurde die Suspension etwa 18 Std. bei 37°C inkubiert. Die DNA wurde durch Extraktion mit Phenol, Phenol-Chloroform-Isoamylalkohol und Chloroform-Isoamylalkohol mittels Standard-Verfahren gereinigt. Dann wurde die DNA durch Zugabe von 1/50 Volumen 3 M Natriumacetat und 2 Volumina Ethanol, anschließender Inkubation für 30 min bei - 20°C und 30 min Zentrifugation bei 12000 U/min in einer Hochgeschwindigkeitszentrifuge mit einem SS34-Rotor (Sorvall) gefällt. Die DNA wurde in 1 ml TE-Puffer gelöst, der 20 µg/ml RNase A enthielt, und für mindestens 3 Std. bei 4°C gegen 1000 ml TE-Puffer dialysiert. Während dieser Zeit wurde der Puffer 3mal ausgetauscht. Zu Aliquots von 0,4 ml der dialysierten DNA-Lösung wurden 0,4 ml 2 M LiCl und 0,8 ml Ethanol zugegeben. Nach 30 min Inkubation bei -20°C wurde die DNA durch Zentrifugation gesammelt (13000 U/min, Biofuge Fresco, Heraeus, Hanau, Deutschland). Das DNA-Pellet wurde in TE-Puffer gelöst. Durch dieses Verfahren hergestellte DNA konnte für alle Zwecke verwendet werden, einschließlich Southern-Blotting oder zur Konstruktion genomischer Banken.

### Beispiel 2: Konstruktion genomischer Corynebacterium glutamicum (ATCC13032)-Banken in Escherichia coli

Ausgehend von DNA, hergestellt wie in Beispiel 1 beschrieben, wurden gemäß bekannter und gut eingeführter Verfahren (siehe bspw. Sambrook, J. et al. (1989) "Molecular Cloning: A Laboratory Manual". Cold Spring Harbor Laboratory Press oder Ausubel, F.M. et al. (1994) "Current Protocols in Molecular Biology", John Wiley & Sons) Cosmid- und Plasmid-Banken hergestellt.

Es ließ sich jedes Plasmid oder Cosmid einsetzen. Besondere Verwendung fanden die Plasmide pBR322 (Sutcliffe, J.G. (1979) Proc. Natl Acad. Sci. USA, 75: 3737-3741); pACYC177 (Change & Cohen (1978) J. Bacteriol. 134: 1141-1156); Plasmide der pBS-Reihe (pBSSK+, pBSSK- und andere; Stratagene, LaJolla, USA) oder Cosmide, wie SuperCosl (Stratagene, LaJolla, USA) oder Lorist6 (Gibson, T.J. Rosenthal, A., und Waterson, R.H. (1987) Gene 53: 283-286.

### Beispiel 3: DNA-Sequenzierung und Computer-Funktionsanalyse

Genomische Banken, wie in Beispiel 2 beschrieben, wurden zur DNA-Sequenzierung gemäß Standard-Verfahren, insbesondere dem Kettenabbruchverfahren mit AB1377-Sequenziermaschinen (s. z.B. Fleischman, R.D. et al. (1995) "Whole-genome Random Sequencing and Assembly of Haemophilus Influenzae Rd., Science 269; 496-512) verwendet. Die Sequenzierprimer mit den folgenden Nukleotidsequenzen wurden verwendet: 5'-GGAAACAGTATGACCATG-3' oder 5'-GTAAAACGACGGCCAGT-3'.

### Beispiel 4: In-vivo-Mutagenese

In vivo-Mutagenese von *Corynebacterium glutamicum* kann durchgeführt werden, indem eine Plasmid- (oder andere Vektor-) DNA durch *E*. *coli* oder andere Mikroorganismen (z.B. *Bacillus* spp. oder Hefen, wie *Saccharomyces cerevisiae*) geleitet wird, die die Integrität ihrer genetischen Information nicht aufrechterhalten können. Übliche Mutatorstämme weisen Mutationen in den Genen für das DNA-Reparatursystem auf (z.B., mutHLS, mutD, mutT, usw., zum Vergleich siehe Rupp, W.D. (1996) DNA repair mechanisms in Escherichia coli and Salmonella, S. 2277-2294, ASM: Washington). Diese Stämme sind dem Fachmann bekannt. Die Verwendung dieser Stämme ist bspw. in Greener, A. und Callahan, M. (1994) Strategies 7; 32-34 veranschaulicht.

### Beispiel 5: DNA-Transfer zwischen Escherichia coli und Corynebacterium glutamicum

Mehrere *Corynebacterium-* und *Brevibacterium*-Arten enthalten endogene Plasmide (wie bspw. pHM1519 oder pBL1) die autonom replizieren (für einen Überblick siehe bspw. Martin, J.F. et al. (1987) Biotechnology 5: 137-146). Shuttle-Vektoren für *Escherichia coli* und *Corynebacterium glutamicum* lassen sich leicht mittels Standard-Vektoren für *E*. *coli* konstruieren (Sambrook, J. et al., (1989), "Molecular Cloning: A Laboratory Manual", Cold Spring Harbor Laboratory Press oder Ausubel, F.M. et al. (1994) "Current Protocols in Molecular Biology", John Wiley & Sons), denen ein Replikationsursprung für und ein geeigneter Marker aus *Corynebacterium glutamicum* beigegeben wird. Solche Replikationsursprünge werden vorzugsweise von endogenen Plasmiden entnommen, die aus *Corynebacterium*- und *Brevibactertium*-Arten isoliert worden sind. Besondere Verwendung als Transformationsmarker für diese Arten sind Gene für Kanamycin-Resistenz (wie solche, die vom Tn5- oder Tn-903-Transposon stammen) oder für Chloramphenicol (Winnacker, E.L. (1987) "From Genes to Clones - Introduction to Gene Technology, VCH, Weinheim). Es gibt zahlreiche Beispiele in der Literatur zur Herstellung einer großen Vielzahl von Shuttle-Vektoren, die in *E. coli* und *C*. *glutamicum* repliziert werden, und die für verschiedene Zwecke verwendet werden können, einschließlich Gen-Überexpression (siehe bspw. Yoshihama, M. et al. (1985) J. Bacteriol. 162: 591-597, Martin, J.F. et al., (1987) Biotechnology, 5: 137-146 und Eikmanns, B.J. et al. (1992) Gene 102: 93-98).

Mittels Standard-Verfahren ist es möglich, ein Gen von Interesse in einen der vorstehend beschriebenen Shuttle-Vektoren zu klonieren und solche Hybrid-Vektoren in *Corynebacterium glutamicum-*Stämme einzubringen. Die Transformation von *C. glutamicum* läßt sich durch Protoplastentransformation (Kastsumata, R. et al., (1984) J. Bacteriol. 159, 306-311), Elektroporation (Liebl, E. et al., (1989) FEMS Microbiol. Letters, 53: 399-303) und in Fällen, bei denen spezielle Vektoren verwendet werden, auch durch Konjugation erzielen (wie z.B. beschrieben in Schäfer, A., et (1990) J. Bacteriol. 172: 1663-1666). Es ist ebenfalls möglich, die Shuttle-Vektoren für *C*. *glutamicum* auf *E*. *coli* zu übertragen, indem Plasmid-DNA aus *C*. *glutamicum* (mittels im Fachgebiet bekannter Standard-Verfahren) präpariert wird und in *E*. *coli* transformiert wird. Dieser Transformationsschritt kann mit Standard-Verfahren erfolgen, jedoch wird vorteilhafterweise ein Mcr-defizienter E. coli-Stamm verwendet, wie NM522 (Gough & Murray (1983) J. Mol. Biol. 166: 1-19).

### Beispiel 6: Bestimmung der Expression des mutanten Proteins

Die Beobachtungen der Aktivität eines mutierten Proteins in einer transformierten Wirtszelle beruhen auf der Tatsache, daß das mutante Protein auf ähnliche Weise und in ähnlicher Menge exprimiert wird wie das Wildtyp-Protein. Ein geeignetes Verfahren zur Bestimmung der Transkriptionsmenge des mutanten Gens (ein Anzeichen für die mRNA-Menge, die für die Translation des Genprodukts verfügbar ist) ist die Durchführung eines Northern-Blots (s. bspw. Ausubel et al., (1988) Current Protocols in Molecular Biology, Wiley: New York), wobei ein Primer, der so ausgestaltet ist, daß er an das Gen von Interesse bindet, mit einer nachweisbaren (gewöhnlich radioaktiven oder chemilumineszierenden) Markierung versehen wird, so daß - wenn die Gesamt-RNA einer Kultur des Organismus extrahiert, auf einem Gel aufgetrennt, auf eine stabile Matrix übertragen und mit dieser Sonde inkubiert wird - die Bindung und die Quantität der Bindung der Sonde das Vorliegen und auch die Menge von mRNA für dieses Gen anzeigt. Diese Information ist ein Nachweis für das Ausmaß der Transkription des mutanten Gens. Gesamt-Zell-RNA läßt sich durch verschiedene Verfahren aus *Corynebacterium glutamicum* isolieren, die im Fachgebiet bekannt sind, wie beschrieben in Bormann, E.R. et al., (1992) Mol. Microbiol. 6: 317-326.

Zur Bestimmung des Vorliegens oder der relativen Menge von Protein, das aus dieser mRNA translatiert wird, können Standard-Techniken, wie Western-Blot, eingesetzt werden (s. bspw. Ausubel et al. (1988) "Current Protocols in Molecular Biology", Wiley, New York). Bei diesem Verfahren werden Gesamt-Zellproteine extrahiert, durch Gelelektrophorese getrennt, auf eine Matrix, wie Nitrocellulose, übertragen und mit einer Sonde, wie einem Antikörper, inkubiert, die an das gewünschte Protein spezifisch bindet. Diese Sonde ist gewöhnlich mit einer chemilumineszierenden oder kolorimetrischen Markierung versehen, die sich leicht nachweisen läßt. Das Vorliegen und die beobachtete Menge an Markierung zeigt das Vorliegen und die Menge des gesuchten Mutantenproteins in der Zelle an.

### Beispiel 7: Wachstum von genetisch verändertem Corynebacterium glutamicum - Medien und Anzuchtbedingungen

Genetisch veränderte Corynebakterien werden in synthetischen oder natürlichen Wachstumsmedien gezüchtet. Eine Anzahl unterschiedlicher Wachstumsmedien für Corynebakterian sind bekannt und leicht erhältlich (Lieb et al. (1989) Appl. Microbiol. Biotechnol. 32: 205-210; von der Osten et al. (1998) Biotechnology Letters 11: 11-16; Patent DE 4 120 867; Liebl (1992) "The Genus Corynebacterium", in: The Procaryotes, Bd. II, Balows, A., et al., Hrsg. Springer-Verlag). Diese Medien bestehen aus einer oder mehreren Kohlenstoffquellen, Stickstoffquellen, anorganischen Salzen, Vitaminen und Spurenelementen. Bevorzugte Kohlenstoffquellen sind Zucker, wie Mono-, Di- oder Polysaccharide. Sehr gute Kohlenstoffquellen sind bspw. Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte aus der Zucker-Raffinierung zu den Medien geben. Es kann auch vorteilhaft sein, Gemische verschiedener Kohlenstoffquellen zuzugeben. Andere mögliche Kohlenstoffquellen sind Alkohole und organische Säuren, wie Methanoi, Ethanol, Essigsäure oder Milchsäure. Stickstoffquellen sind gewöhnlich organische oder anorganische Stickstoffverbindungen oder Materialien, die diese Verbindungen enthalten. Beispielhafte Stickstoffquellen umfassen Ammoniak-Gas oder Ammoniumsalze, wie NH₄Cl oder (NH₄)₂SO₄, NH₄OH, Nitrate, Harnstoff, Aminosäuren oder komplexe Stickstoffquellen, wie Maisquellwasser, Sojamehl, Sojaprotein, Hefeextrakte, Fleischextrakte und andere.

Anorganische Salzverbindungen, die in den Medien enthalten sein können, umfassen die Chlorid-, Phosphor-, oder Sulfatsalze von Calcium, Magnesium, Natrium, Kobalt, Molybdän, Kalium, Mangan, Zink, Kupfer und Eisen. Chelatbildner können zum Medium gegeben werden, um die Metallionen in Lösung zu halten. Besonders geeignete Chelatbildner umfassen Dihydroxyphenole, wie Catechol oder Protocatechuat oder organische Säuren, wie Citronensäure. Die Medien enthalten üblicherweise auch andere Wachstumsfaktoren, wie Vitamine oder Wachstumsförderer, zu denen bspw. Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Die genaue Zusammensetzung der Medienverbindungen hängt stark vom jeweiligen Experiment ab und wird für jeden Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten sind sterilisiert, entweder durch Hitze (20 min bei 1,5 bar und 121_C) oder durch Sterilfiltration. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Anzuchtbedingungen werden für jedes Experiment gesondert definiert. Die Temperatur sollte zwischen 15°C und 45°C liegen und kann während des Experimentes konstant gehalten oder verändert werden. Der pH-Wert des Mediums sollte im Bereich von 5 bis 8,5, vorzugsweise um 7,0 liegen, und kann durch Zugabe von Puffern zu den Medien aufrechterhalten werden. Ein beispielhafter Puffer für diesen Zweck ist ein Kaliumphosphatpuffer. Synthetische Puffer, wie MOPS, HEPES; ACES usw., können alternativ oder gleichzeitig verwendet werden. Der Anzucht-pH-Wert läßt sich während der Anzucht auch durch Zugabe von NaOH oder NHaOH konstant halten. Werden komplexe Medienkomponenten, wie Hefe-Extrakt verwendet, sinkt der Bedarf an zusätzlichen Puffern, da viele komplexe Verbindungen eine hohe Pufferkapazität aufweisen. Beim Einsatz eines Fermenters für die Anzucht von Mikroorganismen kann der pH-Wert auch mit gasförmigem Ammoniak reguliert werden.

Die Inkubationsdauer liegt gewöhnlich in einem Bereich von mehreren Stunden bis zu mehreren Tagen. Diese Zeit wird so ausgewählt, daß sich die maximale Menge Produkt in der Brühe ansammelt. Die offenbarten Wachstumsexperimente können in einer Vielzahl von Behältern, wie Mikrotiterplatten, Glasröhrchen, Glaskolben oder Glas- oder Metallfermentern unterschiedlicher Größen durchgeführt werden. Zum Screening einer großen Anzahl von Klonen sollten die Mikroorganismen in Mikrotiterplatten, Glasröhrchen oder Schüttelkolben entweder mit oder ohne Schikanen gezüchtet werden. Vorzugsweise werden 100-ml-Schüttelkolben verwendet, die mit 10% (bezogen auf das Volumen) des erforderlichen Wachstumsmediums gefüllt sind. Die Kolben sollten auf einem Kreiselschüttler (Amplitude 25 mm) mit einer Geschwindigkeit im Bereich von 100-300 U/min geschüttelt werden. Verdampfungsverluste können durch Aufrechterhalten einer feuchten Atmosphäre verringert werden; alternativ sollte für die Verdampfungsverluste eine mathematische Korrektur durchgeführt werden.

Werden genetisch modifizierte Klone untersucht, sollten auch ein unmodifizierter Kontrollklon oder ein Kontrollklon getestet werden, der das Basisplasmid ohne Insertion enthält. Das Medium wird auf eine OD₆₀₀ von 0,5 - 1,5 angeimpft, wobei Zellen verwendet werden, die auf Agarplatten gezüchtet wurden, wie CM-Platten (10 g/l Glucose, 2,5 g/l NaCl, 2 g/l Harnstoff, 10 g/l Polypepton, 5 g/l Hefeextrakt, 5 g/l Fleischextrakt, 22 g/l Agar pH-Wert 6,8 mit 2 M NaOH), die bei 30°C inkubiert worden sind. Das Animpfen der Medien erfolgt entweder durch Einbringen einer Kochsalzlösung von *C*. *glutamicum-Zellen* von CM-Platten oder durch Zugabe einer flüssigen Vorkultur dieses Bakteriums.

### Beispiel 8: In-vitro-Analyse der Funktion mutanter Proteine

Die Bestimmung der Aktivitäten und kinetischen Parameter von Enzymen ist im Fachgebiet gut bekannt. Experimente zur Bestimmung der Aktivität eines bestimmten veränderten Enzyms müssen an die spezifische Aktivität des Wildtypenzyms angepaßt werden, was innerhalb der Fähigkeiten des Fachmann liegt. Überblicke über Enzyme im Allgemeinen sowie spezifische Einzetherten, die die Strukture, Kinetiken, Prinzipien, Verfahren, Anwendungen und Beispiele zur Bestimmung vieler Enzymaktivitäten betreffen, können bspw. in den nachstehenden Literaturstellen gefunden werden: Dixon, M., und Webb, E.C: (1979) Enzymes, Longmans, London; Fersht (1985) Enzyme Structure and Mechanism, Freeman, New York; Walsh (1979) Enzymatic Reaction Mechanisms. Freeman, San Francisco; Price, N.C., Stevens, L. (1982) Fundamentals of Enzymology. Oxford Univ. Press: Oxford; Boyer, P.D: Hrsg. (1983) The Enzymes, 3. Aufl. Academic Press, New York; Bisswanger, H. (1994) Enzymkinetik, 2. Aufl. VCH, Weinheim (ISBN 3527300325); Bergmeyer, H.U., Bergmeyer, J., Graßl, M. Hrsg. (1983-1986) Methods of Enzymatic Analysis, 3. Aufl. Bd. I-XII, Verlag Chemie: Weinheim; und Ullmann's Encyclopedia of Industrial Chemistry (1987) Bd. A9, "Enzymes", VCH, Weinheim, S. 352-363.

Die Aktivität von Proteinen, die an DNA binden, kann durch viele gut eingeführte Verfahren gemessen werden, wie DNA-Banden-Shift-Assays (die auch als Gelretardations-Assays bezeichnet werden). Die Wirkung dieser Proteine auf die Expression anderer Moleküle kann mit Reportergenassays (wie beschrieben in Kolmar, H. et al., (1995) EMBO J. 14: 3895-3904 und den darin zitierten Literaturstellen) gemessen werden. Reportergen-Testsysteme sind wohlbekannt und für Anwendungen in pro- und eukaryotischen Zellen etabliert, wobei Enzyme, wie beta-Galactosidase, Grün-Fluoreszenz-Protein und mehrere andere verwendet werden.

Die Bestimmung der Aktivität von Membran-Transportproteinen kann gemäß den Techniken, wie beschrieben in Gennis, R.B. (1989) "Pores, Channels and Transporters", in Biomembranes, Molecular Structure and Function, Springer: Heidelberg, S. 85-137; 199-234; und 270-322, erfolgen.

### Beispiel 9: Analyse des Einflusses von mutiertem Protein auf die Produktion des gewünschten Produktes

Die Wirkung der genetischen Modifikation in *C*. *glutamicum* auf die Produktion einer gewünschten Verbindung (wie einer Aminosäure) kann bestimmt werden, indem die modifizierten Mikroorganismen unter geeigneten Bedingungen (wie solchen, die vorstehend beschrieben sind) gezüchtet werden und das Medium und/oder die zellulären Komponenten auf die erhöhte Produktion des gewünschten Produktes (d.h. einer Aminosäure) untersucht wird. Solche Analysetechniken sind dem Fachmann wohlbekannt und umfassen Spektroskopie, Dünnschichtchromatographie, Färbeverfahren verschiedener Art, enzymatische und mikrobiologische Verfahren sowie analytische Chromatographie, wie Hochleistungs-Flüssigkeitschromatographie (s. bspw. Ullman, Encyclopedia of Industrial Chemistry, Bd. A2, S. 89-90 und S. 443-613, VCH: Weinheim (1985); Fallon, A., et al., (1987) "Applications of HPLC in Biochemistry" in: Laboratory Techniques in Biochemistry and Molecular Biology, Bd. 17; Rehm et al. (1993) Biotechnology, Bd. 3, Kapitel III: "Product recovery and purification", S. 469-714, VCH: Weinheim; Belter, P.A. et al. (1988) Bioseparations: downstream processing for Biotechnology, John Wiley and Sons; Kennedy, J.F. und Cabral, J.M.S. (1992) Recovery processes for biological Materials, John Wiley and Sons; Shaeiwitz, J.A. und Henry, J.D. (1988) Biochemical Separations, in Ullmann's Encyclopedia of Industrial Chemistry, Bd. B3; Kapitel 11, S. 1-27, VCH: Weinheim; und Dechow, F.J. (1989) Separation and purification techniques in biotechnology, Noyes Publications).

Zusätzlich zur Messung des Fermentationsendproduktes ist es ebenfalls möglich, andere Komponenten der Stoffwechselwege zu analysieren, die zur Produktion der gewünschten Verbindung verwendet werden, wie Zwischen- und Nebenprodukte, um die Gesamt-Produktivität des Organismus, die Ausbeute und/oder die Effizienz der Produktion der Verbindung zu bestimmen. Die Analyseverfahren umfassen Messungen der Nährstoffmengen im Medium (bspw. Zucker, Kohlenwasserstoffe, Stickstoffquellen, Phosphat und andere Ionen), Messungen der Biomassezusammensetzung und des Wachstums, Analyse der Produktion gewöhnlicher Metabolite aus Biosynthesewegen und Messungen von Gasen, die während der Fermentation erzeugt werden. Standard-verfahren für diese Messungen sind in Applied Microbial Physiology; A Practical Approach, P.M. Rhodes und P.F. Stanbury, Hrsg. IRL Press, S. 103-129; 131-163 und 165-192 (ISBN: 0199635773) und den darin angegebenen Literaturstellen beschrieben.

### Beispiel 10: Reinigung des gewünschten Produktes aus C. glutamicum-Kultur

Die Gewinnung des gewünschten Produktes aus *C*. *glutamicum*-Zellen oder aus dem Überstand der vorstehend beschriebenen Kultur kann durch verschiedene, im Fachgebiet bekannte Verfahren erfolgen. Wird das gewünschte Produkt von den Zellen nicht sezerniert, können die Zellen aus der Kultur durch langsame Zentrifugation geerntet werden, die Zellen können durch Standard-Techniken, wie mechanische Kraft oder Ultrabeschallung, lysiert werden. Die Zelltrümmer werden durch Zentrifugation entfernt, und die Überstandsfraktion, die die löslichen Proteine enthält, wird zur weiteren Reinigung der gewünschten Verbindung erhalten. Wird das Produkt von den *C*. *glutamicum-*Zellen sezerniert, werden die Zellen durch langsame Zentrifugation aus der Kultur entfernt, und die Überstandsfraktion wird zur weiteren Reinigung behalten.

Die Überstandsfraktion aus beiden Reinigungsverfahren wird einer Chromatographie mit einem geeigneten Harz unterworfen, wobei das gewünschte Molekül entweder auf dem Chromatographieharz zurückgehalten wird, viele Verunreinigungen in der Probe jedoch nicht, oder wobei die Verunreinigungen auf dem Harz zurückbleiben, die Probe hingegen nicht. Diese Chromatographieschritte können nötigenfalls wiederholt werden, wobei die gleichen oder andere Chromatographieharze verwendet werden. Der Fachmann ist in der Auswahl der geeigneten Chromatographieharze und der wirksamsten Anwendung für ein bestimmtes, zu reinigendes Molekül bewandert. Das gereinigte Produkt kann durch Filtration oder Ultrafiltration konzentriert und bei einer Temperatur aufbewahrt werden, bei der die Stabilität des Produktes maximal ist.

Im Fachgebiet sind viele Reinigungsverfahren bekannt, die nicht auf das vorhergehende Reinigungsverfahren eingeschränkt sind. Diese sind bspw. beschrieben in Bailey, J.E. & Ollis, D.F. Biochemical Engineering Fundamentals, McGraw-Hill: New York (1986).

Die Identität und Reinheit der isolierten Verbindungen kann durch Standard-Techniken des Fachgebiets bestimmt werden. Diese umfassen Hochleistungs-Flüssigkeitschromatographie (HPLC), spektroskopische Verfahren, Färbeverfahren, Dünnschichtchromatographie, NIRS, Enzymtest oder mikrobiologische Tests. Diese Analyseverfahren sind zusammengefaßt in: Patek et al. (1994) Appl. Environ. Microbiol. 60: 133-140; Malakhova et al. (1996) Biotekhnologiya 11: 27-32; und Schmidt et al. (1998) Bioprocess Engineer. 19: 67-70. Ulmann's Encyclopedia of Industrial Chemistry (1996) Bd. A27, VCH: Weinheim, S. 89-90, S. 521-540, S. 540-547, S. 559-566, 575-581 und S. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, Bd. 17.

### Äquivalente

Der Fachmann erkennt oder kann - indem er lediglich Routineverfahren verwendet - viele Äquivalente der erfindungsgemäßen spezifischen Ausführungsformen feststellen. Diese Äquivalente sollen von den nachstehenden Patentansprüchen umfaßt sein.

Die Angaben in Tabelle 1 sind folgendermassen zu verstehen:
In Spalte 1 "DNA-ID" bezieht sich die jeweilige Zahl auf die SEQ ID NO des anhängenden Sequenzprotokolls. Eine "5" in der Spalte "DNA-ID" bedeutet demzufolge ein Verweis auf SEQ ID NO:5.
In Spalte 2"AS-ID" bezieht sich die jeweilige Zahl auf die SEQ ID NO des anhängenden Sequenzprotokolls. Eine "6" in der Spalte "AS-ID" bedeutet demzufolge ein Verweis auf SEQ ID NO:6.
In Spalte 3"Identifikation" wird eine eindeutige interne Bezeichnung für jede Sequenz aufgeführt.
In Spalte 4 "AS-POS" bezieht sich die jeweilige Zahl auf die Aminosäureposition der Polypeptidsequenz "AS-ID" in der gleichen Zeile. Eine "26" in der Spalte "AS-POS" bedeutet demzufolge die Aminosäureposition 26 der entsprechend angegebenen Polypeptidsequenz. Die Zählung der Position beginnt N-Terminal bei +1.
In Spalte 5 "AS-Wildtyp" bezeichnet der jeweilige Buchstabe die Aminosäure - dargestellt im Ein-Buchstaben-Code- an der in Spalte 4 angegebenen Position beim entsprechenden Wildtyp-Stamm.
In Spalte 6 "AS-Mutante" bezeichnet der jeweilige Buchstabe die Aminosäure - dargestellt im Ein-Buchstaben-Code- an der in Spalte 4 angegebenen Position beim entsprechenden Mutanten-Stamm.
In Spalte 7"Funktion" wird die physiologische Funktion der entsprechenden Polypeptidsequenz aufgeführt.

Ein-Buchstaben-Code der proteinogenen Aminosäuren:
- A: Alanin
- C: Cystein
- D: Aspartat
- E: Glutamat
- F: Phenylalanin
- G: Glycin
- H: His
- I: Isoleucin
- K: Lysin
- L: Leucin
- M: Methionin
- N: Asparagin
- P: Prolin
- Q: Glutamin
- R: Arginin
- S: Serin
- T: Threonin
- V: Valin
- W: Tryptophan
- Y: Tyrosin

## Patentansprüche

1. Isoliertes Nukleinsäuremolekül codierend für ein Polypeptid mit der Aminosäuresequenz SEQ ID 2 wobei das Nukleinsäuremolekül in der Aminosäureposition 243 die Aminosäure T codiert.

2. Ein Vektor, der wenigstens eine Nukleinsäuresequenz nach Anspruch 1 enthält.

3. Eine prokaryontische Wirtszelle, die mit wenigstens einem Vektor nach Anspruch 2 transfiziert ist.

4. Eine Wirtszelle nach Anspruch 3, wobei die Expression des besagten Nukleinsäuremoleküls zur Verbesserung der Produktion einer Aminosäure aus besagter Zelle führt.

5. Verfahren zur Herstellung einer Aminosäure welches die Kultivierung einer prokaryontischen Zelle beinhaltet, die mit wenigstens einen Vektor nach Anspruch 2 transfiziert worden ist, so dass die Aminosäure produziert wird.

## Claims

1. An isolated nucleic acid molecule, coding for a polypeptide having the amino acid sequence SEQ ID 2, wherein the nucleic acid molecule in the amino acid 243 encodes the amino acid T.

2. A vector, which comprises at least one nucleic acid sequence according to claim 1.

3. A prokaryotic host cell, which is transfected with at least one vector according to claim 2.

4. A host cell according to claim 3, wherein expression of said nucleic acid molecule improves the production of an amino acid from said cell.

5. A method for preparing an amino acid, which comprises culturing a prokaryotic cell which has been transfected with at least one vector according to claim 2 so that the amino acid is produced.

## Revendications

1. Molécule d'acide nucléique isolée codant pour un polypeptide ayant la séquence d'acides aminés SEQ ID 2, la molécule d'acide nucléique codant sur la position d'acide aminé 243 pour l'acide aminé T.

2. Vecteur qui contient au moins une séquence d'acide nucléique selon la revendication 1.

3. Cellule hôte procaryote, qui est transfectée avec au moins un vecteur selon la revendication 2.

4. Cellule hôte selon la revendication 3, l'expression de ladite molécule d'acide nucléique conduisant à une amélioration de la production d'un acide aminé à partir de ladite cellule.

5. Procédé de production d'un acide aminé, qui contient la culture d'une cellule procaryote qui a été transfectée avec au moins un vecteur selon la revendication 2 de façon à produire l'acide aminé.


### SEQUENCE LISTING

<110> BASF SE
<120> Gene die für Glucose-6-Phosphat-Dehydrogenase Proteine codieren
<130> PF00000053030-2 SE/KLC
<140> 07123951.1 <141> 11.11.2002
<160> 2
<170> PatentIn Ver. 2.0
<210> 1
   <211> 1672
   <212> DNA
   <213> Corynebacterium glutamicum
<220>
   <221> 5\222UTR
   <222> (1)..(100)
<220>
   <221> CDS
   <222> (101)..(1645)
<220>
   <221> 3\222UTR
   <222> (1646)..(1672)
<400> 1
   agcacgctgc atcagtaacg gcgacatgaa atcgaattag ttcgatctta tgtggccgtt 60
   acacatcttt cattaaagaa aggatcgtga cactaccatc gtg agc aca aac acg   115
                                               Met Ser Thr Asn Thr
                                               1               5
   acc ccc tcc agc tgg aca aac cca ctg cgc gac ccg cag gat aaa cga   163
   Thr Pro Ser Ser Trp Thr Asn Pro Leu Arg Asp Pro Gln Asp Lys Arg
                   10                  15                  20
   ctc ccc cgc atc gct ggc cct tcc ggc atg gtg atc ttc ggt gtc act   211
   Leu Pro Arg Ile Ala Gly Pro Ser Gly Met Val Ile Phe Gly Val Thr
               25                  30                  35
   ggc gac ttg gct cga aag aag ctg ctc ccc gcc att tat gat cta gca   259
   Gly Asp Leu Ala Arg Lys Lys Leu Leu Pro Ala Ile Tyr Asp Leu Ala
           40                  45                  50
   aac cgc gga ttg ctg ccc cca gga ttc tcg ttg gta ggt tac ggc cgc   307
   Asn Arg Gly Leu Leu Pro Pro Gly Phe Ser Leu Val Gly Tyr Gly Arg
       55                  60                  65
   cgc gaa tgg tcc aaa gaa gac ttt gaa aaa tac gta cgc gat gcc gca   355
   Arg Glu Trp Ser Lys Glu Asp Phe Glu Lys Tyr Val Arg Asp Ala Ala
   70                  75                  80                  85
   agt gct ggt gct cgt acg gaa ttc cgt gaa aat gtt tgg gag cgc ctc   403
   Ser Ala Gly Ala Arg Thr Glu Phe Arg Glu Asn Val Trp Glu Arg Leu
                   90                  95                  100
   gcc gag ggt atg gaa ttt gtt cgc ggc aac ttt gat gat gat gca gct   451
   Ala Glu Gly Met Glu Phe Val Arg Gly Asn Phe Asp Asp Asp Ala Ala
               105                 110                 115
   ttc gac aac ctc gct gca aca ctc aag cgc atc gac aaa acc cgc ggc    499
   Phe Asp Asn Leu Ala Ala Thr Leu Lys Arg Ile Asp Lys Thr Arg Gly
           120                 125                 130
   acc gcc ggc aac tgg gct tac tac ctg tcc att cca cca gat tcc ttc    547
   Thr Ala Gly Asn Trp Ala Tyr Tyr Leu Ser Ile Pro Pro Asp Ser Phe
       135                 140                 145
   aca gcg gtc tgc cac cag ctg gag cgt tcc ggc atg gct gaa tcc acc    595
   Thr Ala Val Cys His Gln Leu Glu Arg Ser Gly Met Ala Glu Ser Thr
   150                 155                 160                 165
   gaa gaa gca tgg cgc cgc gtg atc atc gag aag cct ttc ggc cac aac    643
   Glu Glu Ala Trp Arg Arg Val Ile Ile Glu Lys Pro Phe Gly His Asn
                   170                 175                 180
   ctc gaa tcc gca cac gag ctc aac cag ctg gtc aac gca gtc ttc cca    691
   Leu Glu Ser Ala His Glu Leu Asn Gln Leu Val Asn Ala Val Phe Pro
               185                 190                 195
   gaa tct tct gtg ttc cgc atc gac cac tat ttg ggc aag gaa aca gtt    739
   Glu Ser Ser Val Phe Arg Ile Asp His Tyr Leu Gly Lys Glu Thr Val
           200                 205                 210
   caa aac atc ctg gct ctg cgt ttt gct aac cag ctg ttt gag cca ctg    787
   Gln Asn Ile Leu Ala Leu Arg Phe Ala Asn Gln Leu Phe Glu Pro Leu
       215                 220                 225
   tgg aac tcc aac tac gtt gac cac gtc cag atc acc atg gct gaa gat    835
   Trp Asn Ser Asn Tyr Val Asp His Val Gln Ile Thr Met Ala Glu Asp
   230                 235                 240                 245
   att ggc ttg ggt gga cgt gct ggt tac tac gac ggc atc ggc gca gcc    883
   Ile Gly Leu Gly Gly Arg Ala Gly Tyr Tyr Asp Gly Ile Gly Ala Ala
                   250                 255                 260
   cgc gac gtc atc cag aac cac ctg atc cag ctc ttg gct ctg gtt gcc    931
   Arg Asp Val Ile Gln Asn His Leu Ile Gln Leu Leu Ala Leu Val Ala
               265                 270                 275
   atg gaa gaa cca att tct ttc gtg cca gcg cag ctg cag gca gaa aag    979
   Met Glu Glu Pro Ile Ser Phe Val Pro Ala Gln Leu Gln Ala Glu Lys
           280                 285                 290
   atc aag gtg ctc tct gcg aca aag ccg tgc tac cca ttg gat aaa acc    1027
   Ile Lys Val Leu Ser Ala Thr Lys Pro Cys Tyr Pro Leu Asp Lys Thr
       295                 300                 305
   tcc gct cgt ggt cag tac gct gcc ggt tgg cag ggc tct gag tta gtc    1075
   Ser Ala Arg Gly Gln Tyr Ala Ala Gly Trp Gln Gly Ser Glu Leu Val
   310                 315                 320                 325
   aag gga ctt cgc gaa gaa gat ggc ttc aac cct gag tcc acc act gag    1123
   Lys Gly Leu Arg Glu Glu Asp Gly Phe Asn Pro Glu Ser Thr Thr Glu
                   330                 335                 340
   act ttt gcg gct tgt acc tta gag atc acg tct cgt cgc tgg gct ggt    1171
   Thr Phe Ala Ala Cys Thr Leu Glu Ile Thr Ser Arg Arg Trp Ala Gly
               345                 350                 355
   gtg ccg ttc tac ctg cgc acc ggt aag cgt ctt ggt cgc cgt gtt act   1219
   Val Pro Phe Tyr Leu Arg Thr Gly Lys Arg Leu Gly Arg Arg Val Thr
           360                 365                 370
   gag att gcc gtg gtg ttt aaa gac gca cca cac cag cct ttc gac ggc   1267
   Glu Ile Ala Val Val Phe Lys Asp Ala Pro His Gln Pro Phe Asp Gly
       375                 380                 385
   gac atg act gta tcc ctt ggc caa aac gcc atc gtg att cgc gtg cag   1315
   Asp Met Thr Val Ser Leu Gly Gln Asn Ala Ile Val Ile Arg Val Gln
   390                 395                 400                 405
   cct gat gaa ggt gtg ctc atc cgc ttc ggt tcc aag gtt cca ggt tct   1363
   Pro Asp Glu Gly Val Leu Ile Arg Phe Gly Ser Lys Val Pro Gly Ser
                   410                 415                 420
   gcc atg gaa gtc cgt gac gtc aac atg gac ttc tcc tac tca gaa tcc   1411
   Ala Met Glu Val Arg Asp Val Asn Met Asp Phe Ser Tyr Ser Glu Ser
               425                 430                 435
   ttc act gaa gaa tca cct gaa gca tac gag cgc ctc att ttg gat gcg   1459
   Phe Thr Glu Glu Ser Pro Glu Ala Tyr Glu Arg Leu Ile Leu Asp Ala
           440                 445                 450
   ctg tta gat gaa tcc agc ctc ttc cct acc aac gag gaa gtg gaa ctg   1507
   Leu Leu Asp Glu Ser Ser Leu Phe Pro Thr Asn Glu Glu Val Glu Leu
       455                 460                 465
   agc tgg aag att ctg gat cca att ctt gaa gca tgg gat gcc gat gga   1555
   Ser Trp Lys Ile Leu Asp Pro Ile Leu Glu Ala Trp Asp Ala Asp Gly
   470                 475                 480                 485
   gaa cca gag gat tac cca gcg ggt acg tgg ggt cca aag agc gct gat   1603
   Glu Pro Glu Asp Tyr Pro Ala Gly Thr Trp Gly Pro Lys Ser Ala Asp
                   490                 495                 500
   gaa atg ctt tcc cgc aac ggt cac acc tgg cgc agg cca taa           1645
   Glu Met Leu Ser Arg Asn Gly His Thr Trp Arg Arg Pro
               505                 510                 515
   tttaggggca aaaaatgatc tttgaac                                     1672
<210> 2
   <211> 514
   <212> PRT
   <213> Corynebacterium glutamicum
<400> 2
   Met Ser Thr Asn Thr Thr Pro Ser Ser Trp Thr Asn Pro Leu Arg Asp
     1               5                  10                  15
   Pro Gln Asp Lys Arg Leu Pro Arg Ile Ala Gly Pro Ser Gly Met Val
                    20                  25                  30
   Ile Phe Gly Val Thr Gly Asp Leu Ala Arg Lys Lys Leu Leu Pro Ala
               35                  40                  45
   Ile Tyr Asp Leu Ala Asn Arg Gly Leu Leu Pro Pro Gly Phe Ser Leu
       50                  55                  60
   Val Gly Tyr Gly Arg Arg Glu Trp Ser Lys Glu Asp Phe Glu Lys Tyr
    65                  70                  75                  80
   Val Arg Asp Ala Ala Ser Ala Gly Ala Arg Thr Glu Phe Arg Glu Asn
   85 90 95
   Val Trp Glu Arg Leu Ala Glu Gly Met Glu Phe Val Arg Gly Asn Phe
   100 105 110
   Asp Asp Asp Ala Ala Phe Asp Asn Leu Ala Ala Thr Leu Lys Arg Ile
   115 120 125
   Asp Lys Thr Arg Gly Thr Ala Gly Asn Trp Ala Tyr Tyr Leu Ser Ile
   130 135 140
   Pro Pro Asp Ser Phe Thr Ala Val Cys His Gln Leu Glu Arg Ser Gly
   145 150 155 160
   Met Ala Glu Ser Thr Glu Glu Ala Trp Arg Arg Val Ile Ile Glu Lys
   165 170 175
   Pro Phe Gly His Asn Leu Glu Ser Ala His Glu Leu Asn Gln Leu Val
   180 185 190
   Asn Ala Val Phe Pro Glu Ser Ser Val Phe Arg Ile Asp His Tyr Leu
   195 200 205
   Gly Lys Glu Thr Val Gln Asn Ile Leu Ala Leu Arg Phe Ala Asn Gln
   210 215 220
   Leu Phe Glu Pro Leu Trp Asn Ser Asn Tyr Val Asp His Val Gln Ile
   225 230 235 240
   Thr Met Ala Glu Asp Ile Gly Leu Gly Gly Arg Ala Gly Tyr Tyr Asp
   245 250 255
   Gly Ile Gly Ala Ala Arg Asp Val Ile Gln Asn His Leu Ile Gln Leu
   260 265 270
   Leu Ala Leu Val Ala Met Glu Glu Pro Ile Ser Phe Val Pro Ala Gln
   275 280 285
   Leu Gln Ala Glu Lys Ile Lys Val Leu Ser Ala Thr Lys Pro Cys Tyr
   290 295 300
   Pro Leu Asp Lys Thr Ser Ala Arg Gly Gln Tyr Ala Ala Gly Trp Gln
   305 310 315 320
   Gly Ser Glu Leu Val Lys Gly Leu Arg Glu Glu Asp Gly Phe Asn Pro
   325 330 335
   Glu Ser Thr Thr Glu Thr Phe Ala Ala Cys Thr Leu Glu Ile Thr Ser
   340 345 350
   Arg Arg Trp Ala Gly Val Pro Phe Tyr Leu Arg Thr Gly Lys Arg Leu
   355 360 365
   Gly Arg Arg Val Thr Glu Ile Ala Val Val Phe Lys Asp Ala Pro His
   370 375 380
   Gln Pro Phe Asp Gly Asp Met Thr Val Ser Leu Gly Gln Asn Ala Ile
   385 390 395 400
   Val Ile Arg Val Gln Pro Asp Glu Gly Val Leu Ile Arg Phe Gly Ser
   405 410 415
   Lys Val Pro Gly Ser Ala Met Glu Val Arg Asp Val Asn Met Asp Phe
                  420                  425                  430
   Ser Tyr Ser Glu Ser Phe Thr Glu Glu Ser Pro Glu Ala Tyr Glu Arg
             435                  440                  445
   Leu Ile Leu Asp Ala Leu Leu Asp Glu Ser Ser Leu Phe Pro Thr Asn
        450                  455                  460
   Glu Glu Val Glu Leu Ser Trp Lys Ile Leu Asp Pro Ile Leu Glu Ala
   465                  470                  475                  480
   Trp Asp Ala Asp Gly Glu Pro Glu Asp Tyr Pro Ala Gly Thr Trp Gly
                       485                  490                  495
   Pro Lys Ser Ala Asp Glu Met Leu Ser Arg Asn Gly His Thr Trp Arg
                  500                  505                  510
   Arg Pro